# EUROPEAN PATENT SPECIFICATION

(11) **EP 2 944 626 B1**
(45) Date of publication and mention of the grant of the patent: **10.10.2018**
(21) Application number: 14737825.1
(22) Date of filing: 06.01.2014
(51) Int. Cl.: A61K 49/10, G01N 33/68, C07D 263/56, C07C 235/34, C07D 235/14

(54) **MR IMAGING DIAGNOSTIC AGENT FOR INTRACTABLE NEUROLOGICAL DISEASE**
MR-BILDGEBUNGS-DIAGNOSTIKUM FÜR SCHWER BEHANDELBARE NEUROLOGISCHE ERKRANKUNGEN
AGENT DE DIAGNOSTIC IRM POUR UNE MALADIE NEUROLOGIQUE RÉFRACTAIRE

(30) Priority: 09.01.2013 JP 2013001948; 27.09.2013 JP 2013202531
(43) Date of publication of application: 18.11.2015
(73) Proprietor: Shiga University Of Medical Science, Otsu-shi, Shiga 520-2192 (JP)
(72) Inventor: TOOYAMA, Ikuo, Otsu-shi Shiga 520-2192 (JP); TAGUCHI, Hiroyasu, Otsu-shi Shiga 520-2192 (JP); YANAGISAWA, Daijiro, Otsu-shi Shiga 520-2192 (JP)
(74) Representative: Gevers & Orès
(86) International application number: PCT/JP2014/050005
(87) International publication number: WO 2014/109296

(56) References cited:
- WO-A1-2007/111179
- WO-A1-2010/098502
- JP-A- 2009 067 762
- JP-A- 2009 067 762
- NEUROSCIENCE RESEARCH vol. 63, no. 1, 2009, pages 76 - 81, XP025803704

## Description

### Technical Field

The present invention relates to a diagnostic imaging agent for intractable neurological diseases and, in particular, to a novel styrylbenzoxazole derivative or a salt thereof that is useful for nuclear magnetic resonance (MR) diagnostic imaging for Alzheimer's disease.

### Background Art

Alzheimer's disease is a disease characterized by progressive dementia occurring from presenium to old age, and the number of Japanese patients suffering from the disease is said to be one million or more at present. It is anticipated that this number is certain to increase in the future, in association with the aging population. Progress in research for Alzheimer's disease promotes the active development of basic therapeutic agents. To treat Alzheimer's disease with these new agents, a noninvasive method that accurately diagnoses Alzheimer's disease in an early stage is essential.

Clinical symptoms of Alzheimer's disease are memory disorder, higher brain dysfunction (aphasia, apraxia, agnosia, and constructional apraxia), and the like. The symptoms are often commonly observed in other dementia disorders, and it is very difficult to make a definitive diagnosis of Alzheimer's disease only by clinical symptoms.

On the other hand, histopathological characteristics to determine Alzheimer's disease include senile plaques and neurofibrillary tangles. Main components of the former are amyloid β proteins that have β-sheet structures, and main components of the latter are hyperphosphorylated tau proteins. It is known that in Alzheimer's disease, well before the onset of clinical symptoms, the above pathologic tissue change such as accumulation of aggregated amyloid β proteins starts in the brain. Therefore, detection of aggregated amyloid β proteins as a marker will serve as one method for early diagnosis of the diseases in which amyloids accumulate, especially, Alzheimer's disease.

From such a viewpoint, in recent years, radioactive contrast media have been studied that selectively bind to intracerebral amyloid β proteins for use in positron emission tomography (PET) and single-photon emission computed tomography (SPECT). Examples of classical compounds having a high affinity to amyloids include congo red, thioflavin S, and thioflavin T, which are used in pathologic definitive diagnosis of Alzheimer's disease. It is difficult for many of them to pass through the blood-brain barrier, and even if they are intravenously administered, they rarely move into the brain.

Therefore, contrast media have been studied in consideration of the permeability through the blood-brain barrier, and contrast media such as ISB, PIB (Non-patent Literature 1), BF-168 (Patent Literature 1 and Non-patent Literature 2), and BF-227 (Non-patent Literature 3) have been developed. Some of them have had good results in clinical trials (Non-patent Literature 1 and 3). However, since such contrast media use radionuclides such as ¹¹C, ¹³N, ¹⁵O, and ¹⁸F, there is a concern about adverse reaction due to radiation injuries; in addition, such contrast media are problematic in that a cyclotron facility must be provided nearby, and in that the reagents are very expensive. Therefore, a diagnostic method that does not use radionuclides is desired.

The nuclear magnetic resonance imaging (MRI) method is an example of a diagnostic method that does not use radionuclides. There have been reports that imaging of senile plaques by use of a fluorine nuclear magnetic resonance imaging method (fluorine MR imaging method) was successful (Patent Literature 2 to 5, and Non-patent Literature 4).

### Citation List

### Patent Literature

PTL 1: WO2003/106439
PTL 2: WO2005/042461
PTL 3: WO2007/111179
PTL 4: JP2009-067762A
PTL 5: WO2010/098502

### Non-patent Literature

NPL 1: Klunk WE, Engler H, Nordberg A, et al.: Imaging brain amyloid in Alzheimer's disease with Pittsburgh Compound-B. Ann Neurol, 55, 306-319, 2004.

NPL 2: Okamura N, Suemoto T, Shimadzu H, Suzuki M, Shiomitsu T, Akatsu H, Yamamoto T, Staufenbiel M, Yanai K, Arai H, Sasaki H, Kudo Y, Sawada T: Styrylbenzoxazole derivatives for in vivo imaging of amyloid plaques in the brain. J Neurosci, 24 (10), 2535-41, 2004.

NPL 3: Furukawa K, Okamura N, Tashiro M, Waragai M, Furumoto S, Iwata R, Yanai K, Kudo Y, Arai H: Amyloid PET in mild cognitive impairment and Alzheimer's disease with BF-227: comparison to FDG-PET. J Neurol, 257, 721-727, 2010.

NPL 4: Higuchi M, Iwata N, Matsuba Y, Sato K, Sasamoto K, Saido T: 19F and 1H MRI detection of amylid β plaques in vivo, Nature Neuroscience, 8 (4), 527-533, 2005.

### Summary of Invention

### Technical Problem

Among these, the compounds disclosed in Patent Literature 2 to 4 and Non-patent Literature 4 exhibit low sensitivity. Thus, with such compounds, it is difficult to image senile plaques formed in the brain of mouse models while the mice are alive by using a 7 tesla MR imaging apparatus.

The compound disclosed in Patent Literature 5 exhibits good sensitivity, and succeeds in Aβ imaging in live mice. However, the compound disclosed in Patent Literature 5 is characterized as binding to not only Aβ aggregates, which have β-sheet structures, but also Aβ oligomers that do not have β-sheet structures.

As described above, there has been no reagent that specifically binds to Aβ aggregates, which have β-sheet structures, and that allows for imaging of senile plaques formed in the brain of mouse models while the mice are alive by using a 7 tesla MR imaging apparatus.

Therefore, an object of the present invention is to provide a substance having a high binding specificity to and a high detectivity for amyloid β proteins that have β-sheet structures, the substance being appropriate for an MRI contrast medium for diagnosis of Alzheimer's disease.

### Solution to Problem

The present inventors conducted extensive research to achieve the above object, and found that when improvement is made on the compounds disclosed in Patent Literature 3 and 4, and the polyethylene glycol has a length of not shorter than a certain value, the sensitivity can be notably increased, and senile plaques formed in the brain of mouse models can be imaged while the mice are alive by using a 7 tesla MR imaging apparatus. Further, the inventors found that such an improved compound can be applied to the diagnosis of not only Alzheimer's disease, but also other intractable neurological diseases by replacing the site of binding to amyloid β proteins by residues of compounds that bind to other intracerebral abnormal proteins. The inventors also found that the site where Aβ oligomers are present can be detected by using the compound of the present invention in combination with the compound disclosed in Patent Literature 5, which binds to not only Aβ aggregates, but also Aβ oligomers.

More specifically, the present invention relates to a compound represented by formula (I), formula (XI) or a salt thereof, 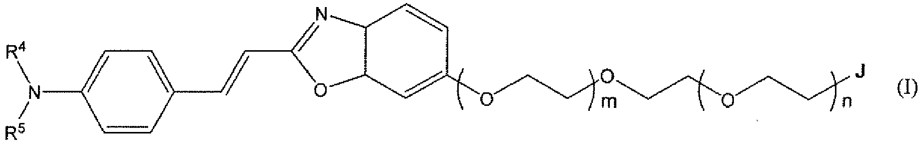 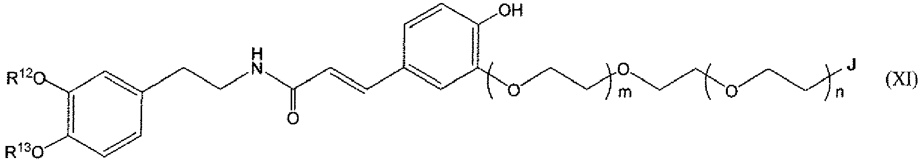 wherein R⁴ and R⁵ represent C₁₋₆ alkyl; R¹² and R¹³ are each independently a hydrogen atom or C₁₋₆ alkyl; m is an integer of 0 to 6; n is an integer of 1 to 5; and J is the following group 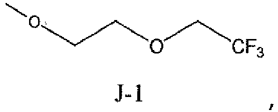 provided that 5 ≤ m + n ≤ 7.
These compounds of formula (I), formula (XI) and salt thereof are compounds falling under the scope of compounds of formula (1) and salt thereof: 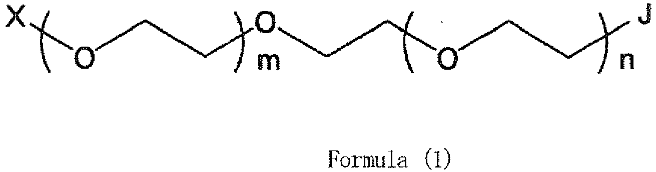 wherein X is a residue of a compound having activity of binding to at least one member selected from the group consisting of amyloid β proteins, tau proteins, α-synuclein, and TDP-43; m is an integer of 0 to 6; n is an integer of 1 to 5; and J is a group selected from the group consisting of 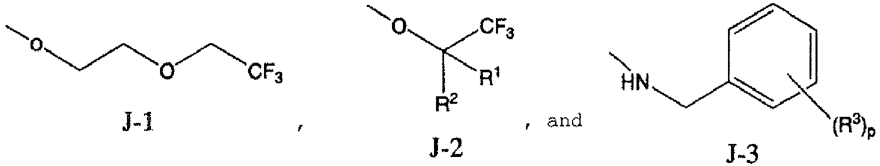 wherein R¹ and R² are each independently a hydrogen atom, a fluorine atom, methyl, or trifluoromethyl; R³ is trifluoromethyl or trifluoromethoxy; and p is an integer of 1 to 4, provided that m + n ≥ 5 when J is J-1, and m + n ≥ 7 when J is J-2 or J-3. The compounds of formula (1) and salts thereof are not all part of the invention, only compounds of formula (I), formula (XI)and salt thereof are part of the invention.

In particular, the present invention relates to a compound represented by formula (I) below or a salt thereof. The compound of formula (I) is a compound of formula (1) wherein X is a residue of a compound having activity of binding to an amyloid β protein. 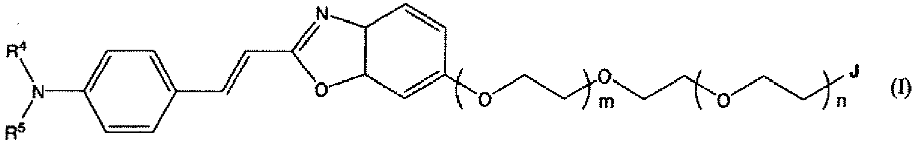 wherein m, n, and J are as described above; and R⁴ and R⁵ represent C₁₋₆ alkyl.

The present invention also relates to a diagnostic imaging agent for a disease in which an amyloid β protein accumulates, the diagnostic imaging agent comprising the compound of formula (I), formula (XI) or a salt thereof as an active ingredient.

The present invention further relates to a staining agent for an amyloid β protein in tissue, including the brain, or a senile plaque in tissue, including the brain, the staining agent comprising the compound of formula (I), formula (XI, or a salt thereof as an active ingredient.

The alkyl groups for R⁴ and R⁵ may be any alkyl groups as long as they are linear or branched C₁₋₆ alkyl groups. Specific examples include methyl, ethyl, propyl, isopropyl, butyl, isobutyl, *tert*-butyl, and the like. The alkyl groups for R⁴ and R⁵ are preferably linear or branched C₁₋₃ alkyl groups.

m is an integer of 0 to 6, and n is an integer of 1 to 5.

The salt of the compound of formula (1), i.e also of formula (I) and formula (XI) may be any salt as long as it is pharmaceutically acceptable. Examples include mineral acid salts, such as hydrochloride, sulfate, nitrate, and phosphate; organic acid salts, such as tartrate, acetate, citrate, malate, fumarate, maleate, benzoate, glycolate, succinate, *p*-toluenesulfonate, and methanesulfonate; and the like. Some of these salts have water of crystallization.

The present invention relates to a diagnostic imaging agent for use in detection of an amyloid β oligomer, the diagnostic imaging agent comprising in combination
(A) the compound of formula (I), formula (XI) or a salt thereof, and
(B) a curcumin derivative represented by formula (2) or a salt thereof,
   formula (2): 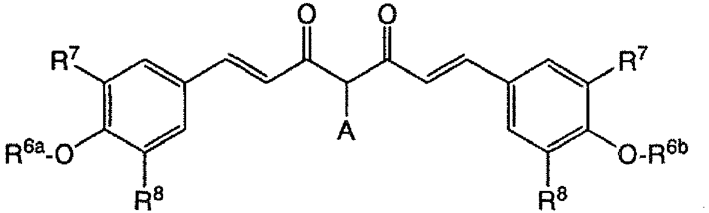 wherein R^{6a} and R^{6b} are each independently a hydrogen atom, alkyl, acetyl, or methoxycarbonyl; R⁷s are each independently a fluorine atom, CHF₂-, CF₃-, CHF₂O-, or CF₃O-; R⁸s are each independently a hydrogen atom or a fluorine atom; A is alkyl, cyano, carboxyl, alkoxycarbonyl, or R⁹-(CH₂)_{q}-; R⁹ is hydroxy, carboxy, cyano, acetyloxy, alkoxycarbonyl, alkoxyalkoxy, hydroxyalkoxy, or CONR¹⁰R¹¹; R¹⁰ and R¹¹ are each independently a hydrogen atom or alkyl; and q is an integer of 1 to 5.

The alkyl groups for R^{6a}, R^{6b}, A, R⁹, R¹⁰, and R¹¹ may be any alkyl groups as long as they are linear or branched C₁₋₆ alkyl groups. The alkyl groups are preferably linear or branched C₁₋₃ alkyl groups. The definition of the alkyl groups is applied to the alkyl groups of alkoxycarbonyl, alkoxyalkoxy, and hydroxyalkoxy in the compound of formula (2).

The salt of the compound of formula (2) may be any salt as long as it is pharmaceutically acceptable. Examples include alkali metal salts, such as potassium salts and sodium salts; alkaline earth metal salts, such as calcium salts; organic amine salts, such as triethanolamine salts and tris(hydroxymethyl)aminomethane salts; and the like. Some of these salts have water of crystallization.

### Advantageous Effects of Invention

The compound of the present invention has high detectivity and is useful as an active ingredient of a diagnostic imaging agent for a disease in which an amyloid β protein accumulates.

In particular, since the compound of the present invention does not bind to amyloid β protein oligomers that do not have β-sheet structures, but specifically binds to amyloid β proteins that have β-sheet structures, it is useful as a diagnostic imaging agent for a disease in which an amyloid β protein that has a β-sheet structure accumulates. Use of the compound of the present invention in combination with the compound disclosed in Patent Literature 5, which binds to not only Aβ aggregates, but also Aβ oligomers, makes it possible to confirm the site where Aβ oligomers are present.

Further, since the compound of the present invention contains many electronically equivalent F atoms, it is useful in particular as an active ingredient of a ¹⁹F-MRI contrast medium. The compound of the present invention is also useful as an active ingredient of a staining agent, for example, as a fluorescent staining agent, for an amyloid β protein in tissue, including the brain, or a senile plaque in tissue, including the brain. Therefore, use of the compound of the present invention makes it possible to perform an early diagnosis of a disease in which an amyloid β protein accumulates, such as Alzheimer's disease.

### Brief Description of Drawings

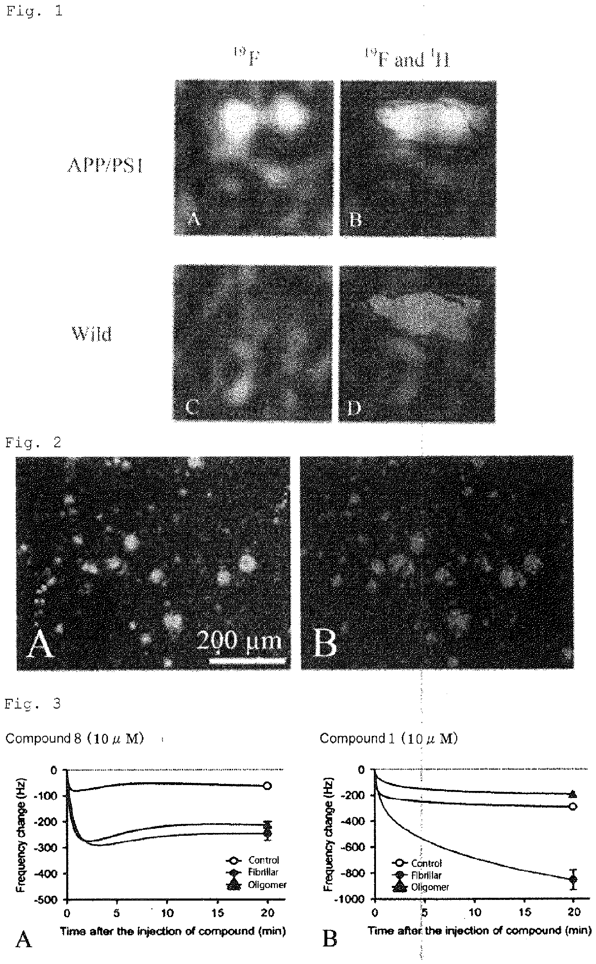

Fig. 1 shows *in vivo* MRI brain images obtained by administering 200 mg/kg of Compound 1 to each of a genetically modified model mouse of Alzheimer's disease (APP/PS1) and a normal wild mouse (Wild) from the tail vein, and performing measurement in a three-hour period, beginning five hours after administration. Figs. 1A and 1C are ¹⁹F-MRI images, and Figs. 1B and 1D show composite photos of ¹⁹F-MRI and ¹H-MRI images.
Fig. 2 shows stain images of a brain slice of the genetically modified model mouse of Alzheimer's disease in Test Example 1. Fig. 2A is a fluorescence image of the compound observed with a DAPI filter set, and Fig. 2B is a β amyloid antibody stain image.
Fig. 3 shows graphs indicating results of analysis of binding property to Aβ oligomers and Aβ fibrils performed using a QCM apparatus in Test Example 3. Fig. 3A shows the frequency change obtained when Compound 8 was added, whereas Fig. 3B shows the frequency change obtained when Compound 1 was added.

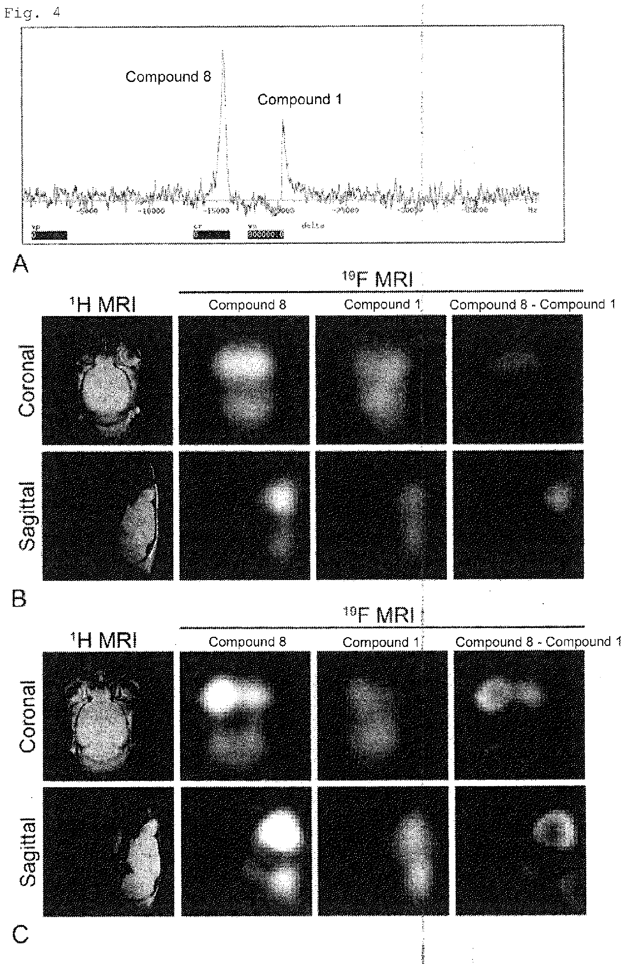

Fig. 4 shows MRI brain images obtained by administering Compound 1 and Compound 8 to APP/PS1 mice from the tail vein and performing measurement in Test Example 4. Fig. 4A shows an NMR spectrum, and Figs. 4B and 4C show, from the left, ¹H-MR images, ¹⁹F-MR images of Compound 8, ¹⁹F-MR images of Compound 1, and images of the ¹⁹F-MR signal difference between Compound 8 and Compound 1. The upper row shows horizontal images, whereas the lower row shows sagittal images. Figs. 4A and 4B show the images obtained from a 16-month-old APP/PS1 mouse, and Fig. 4C shows the images obtained from an 11-month-old APP/PS1 mouse.

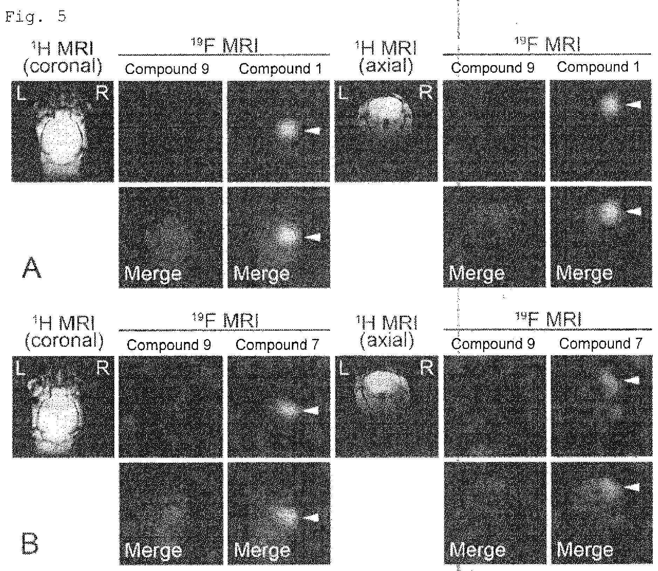

Fig. 5 shows MRI brain images obtained by injecting Compound 1, Compound 7, and Compound 9 into the brain of mice and performing measurement in Test Example 5. Fig. 5A shows MR images of a mouse head in which Compound 9 was injected into the left brain, and Compound 1 was injected into the right brain. Fig. 5B shows MR images of a mouse head in which Compound 9 was injected into the left brain, and Compound 7 was injected into the right brain. The upper row of each figure shows horizontal or coronal axial ¹H MR and ¹⁹F MR images, and the lower row shows combined ¹H MR and ¹⁹F MR images.

### Description of Embodiments

Methods for producing compounds of formula (I) and formula (XI) or salts thereof are described below.

The compound of formula (I) or a salt thereof can be produced by the following method.

The compound of formula (I) wherein J is J-1, J-2, or J-3 can be typically produced by reacting a compound of formula (II) with a compound of formula (III) in the presence of a solvent. 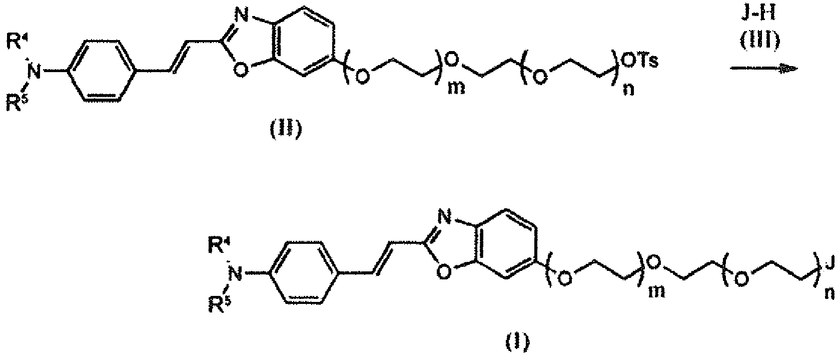 wherein R⁴, R⁵, m, and n are as described above; J is J-1, J-2, or J-3; and Ts is *p*-toluenesulfonyl.

Examples of the solvent used in this reaction include aromatic hydrocarbons, such as benzene, toluene, and xylene; aliphatic hydrocarbons, such as pentane, hexane, heptane, petroleum ether, ligroin, and petroleum benzin; ethers, such as diethyl ether, dipropyl ether, dibutyl ether, tetrahydrofuran, and dioxane; nitriles, such as acetonitrile and propionitrile; acid amides, such as dimethylformamide and dimethylacetamide; sulfoxides, such as dimethyl sulfoxide; sulfones, such as sulfolane; phosphoric amides, such as hexamethylphosphorylamide; halogenated hydrocarbons, such as chloroform, dichloromethane, carbon tetrachloride, and 1,2-dichloroethane; and mixtures thereof.

To promote this reaction, it is desirable to add a base. Examples of the base include organic bases, such as triethylamine, pyridine, *N*-methylmorpholine, 1,8-diazabicyclo[5,4,0]-7-undecene, and *N,N*-dimethylaniline; alkali metals, such as lithium, sodium, and potassium; hydroxides of alkali metals, such as sodium hydroxide and potassium hydroxide; hydroxides of alkaline earth metals, such as calcium hydroxide and barium hydroxide; carbonates of alkali metals, such as lithium carbonate, sodium carbonate, and potassium carbonate; hydrogencarbonates of alkali metals, such as lithium hydrogencarbonate, sodium hydrogencarbonate, and potassium hydrogencarbonate; hydrides of alkali metals, such as lithium hydride, sodium hydride, and potassium hydride; metal alkoxides, such as sodium methoxide, sodium ethoxide, and potassium tert-butoxide; and the like. The base can be used in an amount of 1 to 5 moles, and preferably 1 to 3 moles, per mole of the compound of formula (III).

This reaction can be performed at typically 0 to 150°C, and preferably 20 to 100°C. The reaction time is typically about 1 to about 170 hours.

The compound of formula (III) can be used in an amount of 1 to 10 moles, and preferably 1 to 5 moles, per mole of the compound of formula (II).

Among compounds of formula (II), a compound of formula (II) wherein m = 0 can be produced by a known method (Patent Literature 3), and a compound of formula (II) wherein m ≥ 1 can be produced by p-toluenesulfonylating a compound of formula (IV) by a known method. 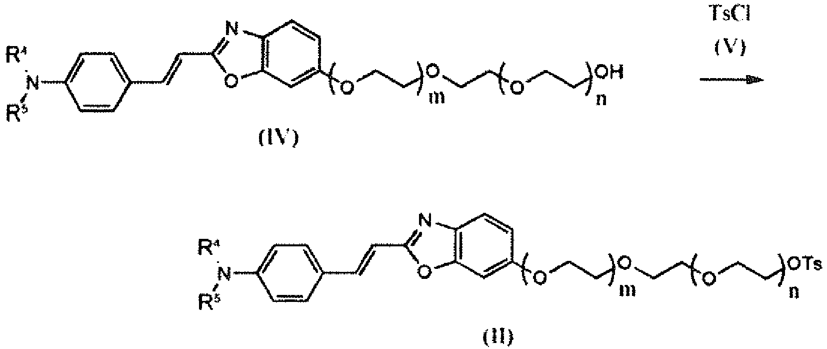 wherein R⁴, R⁵, m, n, and Ts are as described above.

Examples of the solvent used in this reaction include aromatic hydrocarbons, such as benzene, toluene, and xylene; aliphatic hydrocarbons, such as pentane, hexane, heptane, petroleum ether, ligroin, and petroleum benzin; ethers, such as diethyl ether, dipropyl ether, dibutyl ether, tetrahydrofuran, and dioxane; nitriles, such as acetonitrile and propionitrile; acid amides, such as dimethylformamide and dimethylacetamide; sulfoxides, such as dimethyl sulfoxide; halogenated hydrocarbons, such as chloroform, dichloromethane, carbon tetrachloride, and 1,2-dichloroethane; and mixtures thereof.

To promote this reaction, it is desirable to add a base. Examples of the base include organic bases, such as triethylamine, pyridine, *N*-methylmorpholine, 1,8-diazabicycio[5,4,0]-7-undecene, and *N*,*N*-dimethylaniline; carbonates of alkali metals, such as sodium carbonate and potassium carbonate; hydrogencarbonates of alkali metals, such as sodium hydrogencarbonate and potassium hydrogencarbonate; and the like. The base can be used in an amount of 1 to 5 moles, and preferably 1 to 3 moles, per mole of a compound of formula (V).

This reaction can be performed at typically -20 to 100°C, and preferably 0 to 50°C. The reaction time is typically about 1 to about 48 hours.

The compound of formula (V) can be used in an amount of 1 to 10 moles, and preferably 1 to 5 moles, per mole of the compound of formula (IV).

The compound of formula (IV) can be produced by hydrolyzing a compound of formula (VI). For the hydrolysis, an acid is used. 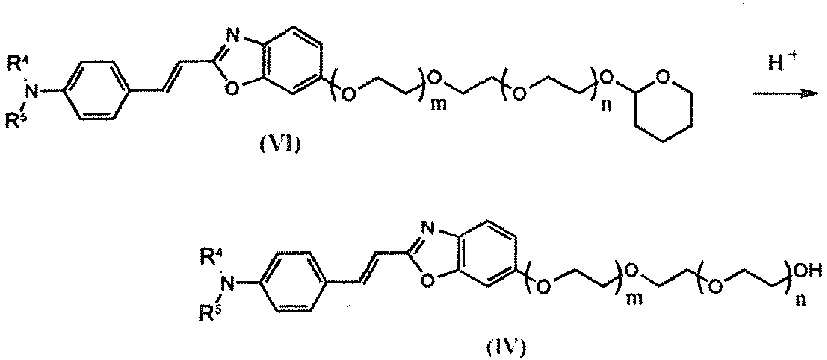 wherein R⁴, R⁵, m, and n are as described above.

Examples of the solvent used in this reaction include water; alcohols, such as methanol, ethanol, propanol, and isopropanol; ethers, such as water-containing tetrahydrofuran and water-containing dioxane; acid amides, such as water-containing dimethylformamide and water-containing dimethylacetamide; nitriles, such as water-containing acetonitrile and water-containing propionitrile; and mixtures thereof.

To promote this reaction, it is desirable to add an acid. Examples of the acid include mineral acids, such as hydrochloric acid, sulfuric acid, nitric acid, and perchloric acid.

This reaction can be performed at typically -20 to 100°C, and preferably 0 to 50°C. The reaction time is typically about 1 to about 50 hours. The acid can be used in an amount of 1 to 20 moles, and preferably 1 to 5 moles, per mole of the compound of (VI).

The compound of formula (VI) can be produced by reacting a compound of formula (VII) with a compound of formula (VIII) . 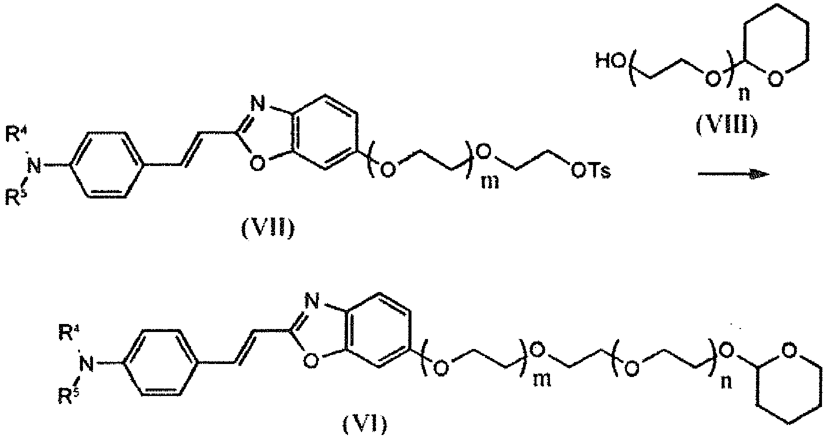 wherein R⁴, R⁵, m, n, and Ts are as described above.

Examples of the solvent used in this reaction include aromatic hydrocarbons, such as benzene, toluene, and xylene; aliphatic hydrocarbons, such as pentane, hexane, heptane, petroleum ether, ligroin, and petroleum benzin; ethers, such as diethyl ether, dipropyl ether, dibutyl ether, tetrahydrofuran, and dioxane; nitriles, such as acetonitrile and propionitrile; acid amides, such as dimethylformamide and dimethylacetamide; sulfoxides, such as dimethyl sulfoxide; sulfones, such as sulfolane; phosphoric amides, such as hexamethylphosphoramide; and mixtures thereof.

To promote this reaction, it is desirable to add a base. Examples of the base include alkali metals, such as lithium, sodium, and potassium; hydrides of alkali metals, such as lithium hydride, sodium hydride, and potassium hydride; metal alkoxides, such as sodium methoxide, sodium ethoxide, and potassium *tert-*butoxide. The base can be used in an amount of 1 to 5 moles, and preferably 1 to 3 moles, per mole of the compound of formula (VIII).

This reaction can be performed at typically -10 to 100°C, and preferably 0 to 60°C. The reaction time is typically about 1 to about 36 hours.

The compound of formula (VIII) can be used in an amount of 1 to 10 moles, and preferably 1 to 5 moles, per mole of the compound of formula (VII).

The compound of formula (VII) can be produced by a known method for producing a similar compound (Patent Literature 3), or a method analogous thereto. For example, the compound of formula (VII) can be typically produced by reacting a compound of formula (IX) with a compound of formula (X) in the presence of triphenylphosphine and diisopropyl azodicarboxylate in a solvent according to the following method. The compound of formula (IX) can be produced by a known method. 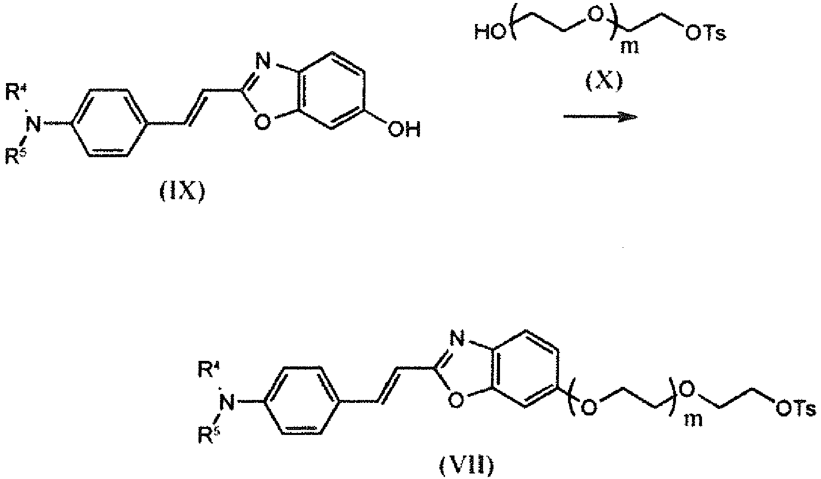 wherein R⁴, R⁵, m, and Ts are as described above.

Examples of the solvent used in this reaction include aromatic hydrocarbons, such as benzene, toluene, and xylene; aliphatic hydrocarbons, such as pentane, hexane, heptane, petroleum ether, ligroin, and petroleum benzin; ethers, such as diethyl ether, dipropyl ether, dibutyl ether, tetrahydrofuran, and dioxane; nitriles, such as acetonitrile and propionitrile; acid amides, such as dimethylformamide and dimethylacetamide; sulfoxides, such as dimethyl sulfoxide; sulfones, such as sulfolane; phosphoric amides, such as hexamethylphosphoramide; halogenated hydrocarbons, such as chloroform, dichloromethane, carbon tetrachloride, and 1,2-dichloroethane; and mixtures thereof.

This reaction can be performed at typically -20 to 100°C, and preferably 0 to 50°C. The reaction time is typically about 1 to about 36 hours.

The compound of formula (X) can be used in an amount of 1 to 10 moles, and preferably 1 to 5 moles, per mole of the compound of formula (IX).

The compound of formula (XI) or a salt thereof can be produced by the following method. The compound of formula (XI) is a compound of formula (1) wherein X is a residue of a compound having activity of binding to an amyloid β protein.

The compound of formula (XI) can be typically produced by reacting a compound of formula (XII) with a compound of formula (XIII) in the presence of a solvent. 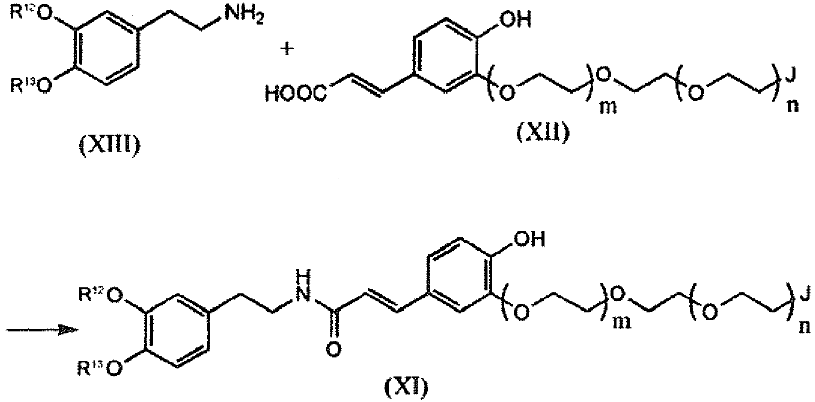 wherein m, n, and J are as described above; and R¹² and R¹³ are each independently a hydrogen atom or C₁₋₆ alkyl.

The alkyl groups for R¹² and R¹³ may be any alkyl groups as long as they are linear or branched C₁₋₆ alkyl groups. The alkyl groups for R¹² and R¹³ are preferably linear or branched C₁₋₃ alkyl groups.

Examples of the solvent used in this reaction include aromatic hydrocarbons, such as benzene, toluene, and xylene; aliphatic hydrocarbons, such as pentane, hexane, heptane, petroleum ether, ligroin, and petroleum benzin; ethers, such as diethyl ether, dipropyl ether, dibutyl ether, tetrahydrofuran, and dioxane; nitriles, such as acetonitrile and propionitrile; acid amides, such as dimethylformamide and dimethylacetamide; sulfoxides, such as dimethyl sulfoxide; halogenated hydrocarbons, such as chloroform, dichloromethane, carbon tetrachloride, and 1,2-dichloroethane; alcohols, such as methanol, ethanol, and propanol; water; and mixtures thereof.

To allow this reaction to proceed, it is desirable to add a dehydration condensing agent. Examples of the condensing agent include diisopropylcarbodiimide, dicyclohexylcarbodiimide, diphenylcarbodiimide, methylpropylcarbodiimide, hexamethylenecarbodiimide, di-*p*-dimethylaminophenylcarbodiimide, 1-t-butyl-3-(3-dimethylamino)propylcarbodiimide, 1-ethyl-3-(3-dimethylamino)propylcarbodiimide, 1-cyclohexyl-3-(3-dimethylamino)propylcarbodiimide, and like 1,3-dialkylcarbodiimides or 1,3-diarylcarbodiimides, and salts thereof. To promote the reaction, it is effective to add an auxiliary agent, such as 1-hydroxybenzotriazole. The condensing agent and the auxiliary agent can be used in an amount of 1 to 5 moles, and preferably 1 to 3 moles, per mole of the compound of formula (XII). Further, when the compound of formula (XIII), which is a starting material, and the carbodiimide of the condensing agent are salts such as hydrochloride, it is necessary to add a base. Examples of the base include organic bases, such as triethylamine, tributylamine, and pyridine. It is desirable that the base is used in an amount sufficient to neutralize the salts of the starting material and the reagent.

This reaction can be performed at typically 0 to 100°C, and preferably 10 to 50°C. The reaction time is about 1 to about 170 hours. The compound of formula (XIII) can be used in an amount of 1 to 5 equivalents, and preferably 1 to 2 equivalents, per mole of the compound (XII).

The compound of formula (XII) can be produced as described below by condensing a compound of formula (XIV) with a compound of formula (XV). 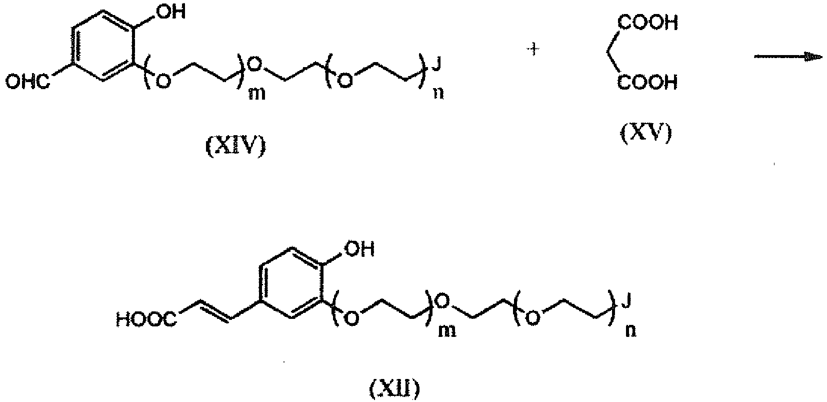 wherein m, n, and J are as described above.

Examples of the solvent used in this reaction include aromatic hydrocarbons, such as benzene, toluene, and xylene; aliphatic hydrocarbons, such as pentane and hexane; ethers, such as diethyl ether and tetrahydrofuran; acid amides, such as dimethylformaldehyde; halogenated hydrocarbons, such as chloroform and dichloromethane; aromatic bases, such as pyridine, 2-picoline, and 4-picoline; alcohols, such as methanol, ethanol, and propanol; water; and mixtures thereof.

To promote this reaction, it is desirable to add a catalyst. Examples of basic catalysts include alkali hydroxides, such as sodium hydroxide and potassium hydroxide; alkali carbonates, such as sodium carbonate and potassium carbonate; metal alkoxides, such as sodium methoxide, sodium ethoxide, and potassium t-butoxide; metal amides, such as sodium amide; organic bases, such as methylamine, ethylamine, aniline, diethylamine, morpholine, piperidine, nicotine, triethylamine, and triethanolamine; and inorganic bases, such as ammonia. Examples of acidic catalysts include acetic anhydride, zinc chloride in glacial acetic acid, and the like. Salts such as ammonium acetate, piperidine acetate, and piperidine benzoate, or amino acids such as glycine and alanine are also effective catalysts. The catalyst can be used in an amount of 0.5 to 5 moles, and preferably 1 to 3 moles, per mole of the compound of formula (XIV).

This reaction is performed at typically 0 to 100°C, and preferably 0 to 50°C. The reaction time is typically 0.5 to 72 hours. The compound of formula (XV) can be used in an amount of 1 to 20 equivalents, and preferably 1 to 10 equivalents, relative to the compound of formula (XIV).

The compound of formula (XIV) can be synthesized by the following steps. 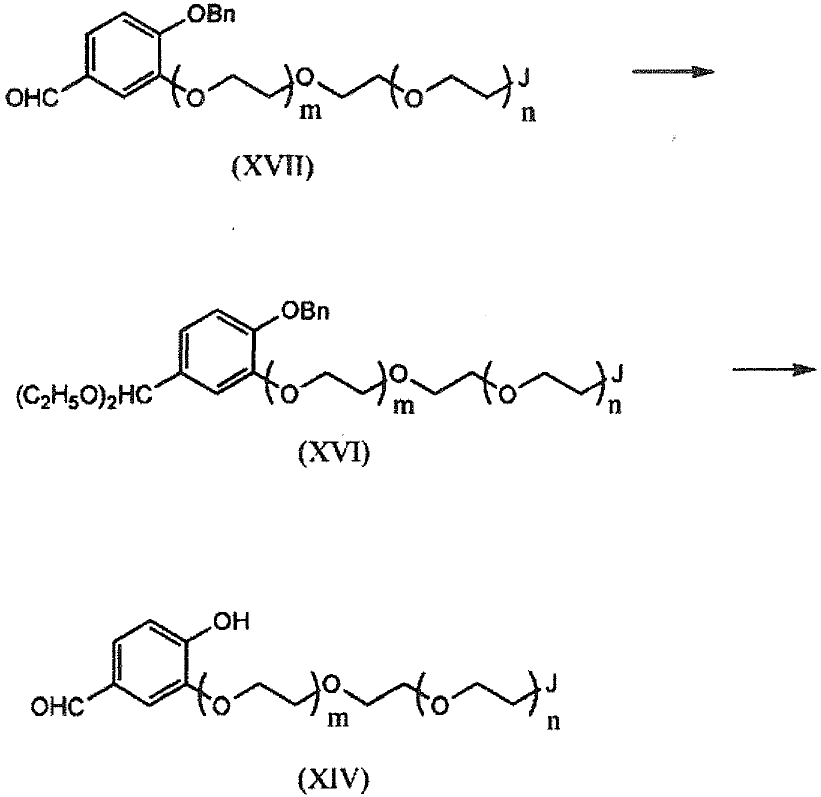 wherein m, n, and J are as described above; and Bn represents benzyl.

First, ethyl orthoformate and a catalytic amount of acid (for example, *p*-toluenesulfonic acid, benzenesulfonic acid, etc.) are added to an ethanol solution of a compound of formula (XVII), and the compound of formula (XVII) is acetalized by a known method, thereby obtaining a compound of (XVI). The obtained acetal was dissolved in methanol and subjected to hydrogenolysis in the presence of a catalyst to perform a debenzylation reaction, thereby obtaining a phenol derivative. This compound is treated with a mineral acid to obtain the compound of formula (XIV).

The compound of formula (XVII) can be obtained by reacting a compound of formula (XIX) with a compound of formula (XVIII). 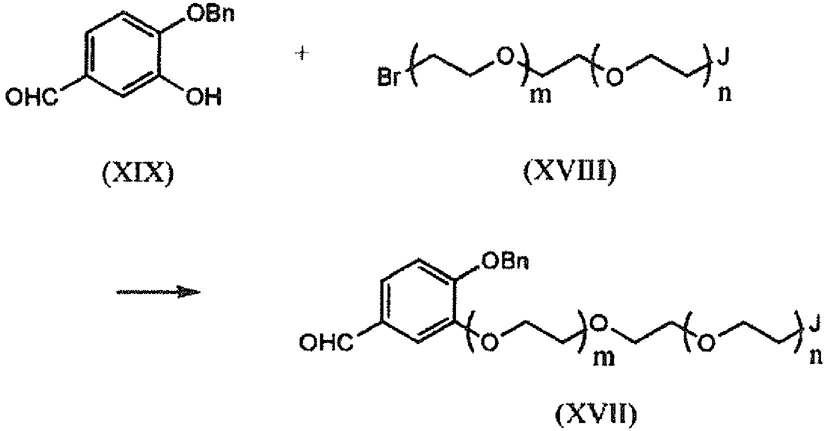 wherein Bn, m, n, and J are as described above.

This reaction is a typical alkylation reaction for phenols, and proceeds easily in the presence of a weak base, for example, an alkali carbonate such as sodium carbonate or potassium carbonate.

The compound of formula (XVIII) can be produced from a compound of formula (XXI) as described below. 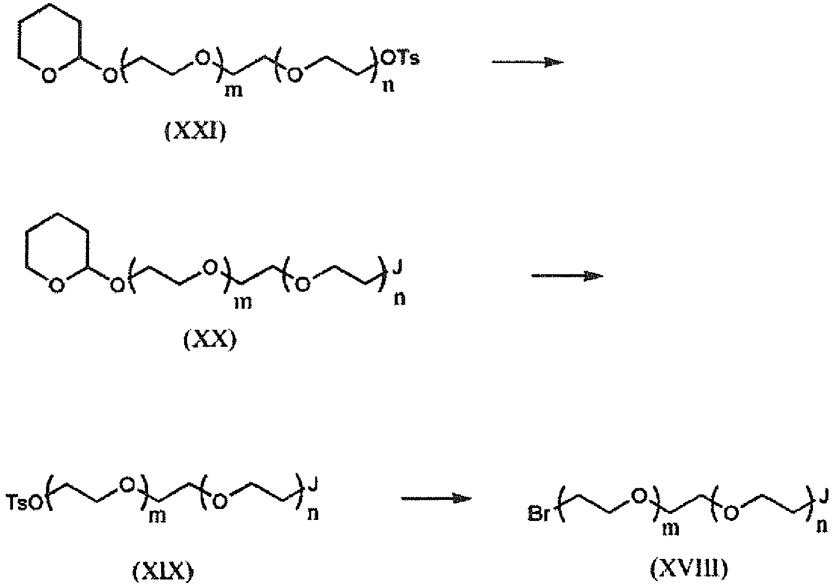 wherein m, n, J, and Ts are as described above.

More specifically, conversion of the compound of formula (XXI) to a compound of formula (XX) is performed by the above-described method for reacting the compound of formula (II) with the compound of formula (III). Subsequently, the compound of formula (XX) is treated with a mineral acid to remove a tetrahydropyranyl protecting group. The resulting alcohol is tosylated to obtain a compound of formula (XIX). This step can be performed by the previously described method (the production method for the compound of formula (II) and the production method for the compound of formula (IV)). The compound of formula (XVIII) can be produced from the compound of formula (XIX) by a known method. More specifically, the compound of formula (XIX) is dissolved in a solvent such as dimethylformamide, and ammonium bromide is allowed to act thereon to replace the tosyloxy group with bromine, thereby obtaining the compound of formula (XVIII).

The compound of formula (I) or (XI) obtained by the production methods described above and a method associated therewith can be isolated and purified by a known means, such as, for example, concentration, vacuum concentration, distillation, fractional distillation, re-dissolution, solvent extraction, crystallization, recrystallization, and chromatography.

In a case where the compound of formula (I) or (XI) is obtained as a free form, it is possible to form a salt by an ordinary method.

Table 1 shows examples of the compound of formula (I) or (XI).

### [Table 1]

**Table 1**

| Compound | Structure | Physical Property |
|---|---|---|
| Compound 1 | 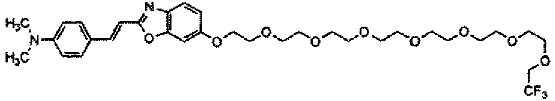 | Oil |
| Compound 2 Not part of the invention | 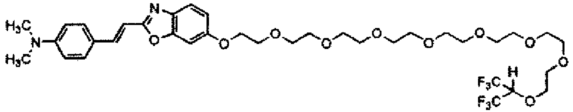 | Oil |
| Compound 3 | 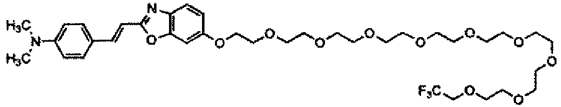 | Oil |
| Compound 4 Not part of the invention | 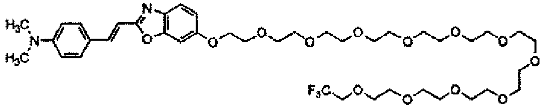 | Oil |
| Compound 5 Not part of the invention | 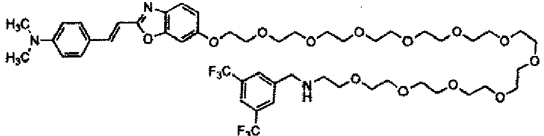 | Oil |
| Compound 6 Not part of the invention | 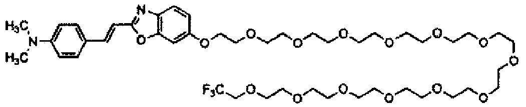 | Oil |
| Compound 7 Not part of the invention | 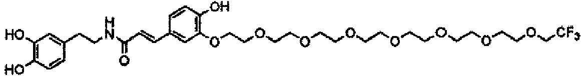 | Oil |

The compound of formula (I) or a salt thereof can be used as a diagnostic imaging agent for a disease in which an amyloid β protein accumulates. The compound of formula (I) or a salt thereof can also be used as a staining agent for an amyloid β protein in tissue, including the brain, or a senile plaque in tissue, including the brain.

Preferred embodiments of the present invention are as follows.
(1) A diagnostic imaging agent for a disease in which an amyloid β protein accumulates, the diagnostic imaging agent comprising the compound of formula (I), formula (XI) or a salt thereof as an active ingredient.
(2) The diagnostic imaging agent according to (1), wherein the disease in which an amyloid β protein accumulates is Alzheimer's disease.
(3) The diagnostic imaging agent according to (1) or (2), wherein diagnostic imaging is MRI.
(4) A staining agent for an amyloid β protein in tissue, including the brain, or a senile plaque in tissue, including the brain, the staining agent comprising the compound of formula (I), formula (XI), or a salt thereof as an active ingredient.
(5) The staining agent according to (4) which is a fluorescent staining agent for the amyloid β protein.
(6) A method for diagnosing a disease caused in full or in part by a β-sheet structure of a protein, the method comprising using the diagnostic imaging agent according to (1).
(7) A method for staining an amyloid β protein in tissue or a senile plaque in tissue, the method comprising using the staining agent according to (4).
(8) A diagnostic imaging agent for use in detection of an amyloid β oligomer, the diagnostic imaging agent comprising (A) the compound of formula (I), formula (XI) or a salt thereof and (B) the compound of formula (2) or a salt thereof in combination.
(9) The diagnostic imaging agent according to (8), wherein diagnostic imaging is MRI.
(10) A method for detecting an amyloid β oligomer, the method comprising using the diagnostic imaging agent according to (8).

Many of the compounds of formula (I) are hydrophobic compounds, and have low solubility in water. As a compound to be administered to a living body, the compound preferably has high solubility in water; among compounds of formula (I), a compound that has a salt is more desirable.

In a case where the compound of formula (I) or a salt thereof is used as a diagnostic imaging agent, it is possible to specifically detect an intracerebral senile plaque by means of the compound. In particular, in a case where an amyloid β protein is noninvasively detected by use of ¹⁹F-MRI, the detectivity depends on the number of fluorine atoms.

The compound of formula (I) binds only to Aβ aggregates, which have β-sheet structures, whereas the compound of formula (2) binds to both Aβ aggregates and Aβ oligomers. Thus, the site where Aβ oligomers are present can be detected by calculating the difference between an image obtained using the compound of formula (I), and an image obtained using the compound of formula (2).

The compound of formula (I), formula (XI) or formula (2), or a salt thereof may be administered locally or systemically when it is used as a diagnostic imaging agent. The administration method is not particularly limited, and the compound or a salt thereof is administered orally or parenterally. The parenteral administration route may be an injection, a drip infusion, or the like under the skin or into the abdominal cavity, vein, artery, or spinal fluid.

The diagnostic imaging agent comprising the compound of formula (I), formula XI, or (2), or a salt thereof is in a pharmaceutically acceptable form appropriate for administration to a human, and contains a physiologically acceptable additive. To such a composition, there may be added, as appropriate, a pharmaceutically acceptable diluent, buffer, solubilizing agent (for example, cyclodextrin, polyethylene glycol, or a surfactant such as Pluronic, Tween, Cremophor, or a phospholipid), soothing agent, etc. The composition may further contain, as necessary, a component such as a pharmaceutically acceptable solvent, stabilizing agent, or antioxidant (for example, ascorbic acid etc.). The dose of the compound of the present invention is suitably selected according to the method of use, the patient's age, sex, and other conditions, and the severity of the disease.

Examples of the disease in which an amyloid β protein accumulates include Down's syndrome in addition to Alzheimer's disease. Examples of the disease caused in full or in part by a β-sheet structure of a protein include frontotemporal dementia, Pick's disease, progressive supranuclear palsy (PSP), prion disease, and the like, in addition to Alzheimer's disease and Down's syndrome.

Examples of the disease in which a tau protein accumulates include Alzheimer's disease, frontotemporal dementia, progressive supranuclear palsy, and the like.

Examples of the disease in which α-synuclein accumulates include Parkinson's disease, Lewy body disease, and the like.

Examples of the disease in which TDP-43 (TAR DNA-binding protein 43 kDa) accumulates include frontotemporal dementia, amyotrophic lateral sclerosis (ALS), and the like.

### Examples

The Synthesis Examples and Test Examples of the present invention are described below; however, the present invention is not limited thereto.

### Synthesis Example 1

### Synthesis of 6-(3',6',9',12',15',18',21'-heptaoxa-23',23',23'-trifluorotricosanyloxy)-2-(4'-dimethylaminostyryl)benzoxazole (Compound 1)

(1) 2-(4-Dimethylaminostyryl)-6-hydroxybenzoxazole (430 mg, 1.53 mmol), triphenylphosphine (960 mg, 3.67 mmol), and 17-(4'-p-toluenes-ulfonyloxy)-3,6,9,12,15-pentaoxaheptadecan-1-ol (1.60 g, 3.67 mmol) were dissolved in DMF (10 mL), and under ice-cooling, the 1.9M toluene solution of isopropyl azodicarboxylate was added dropwise in an amount of 2.25 mL while stirring. After the dropwise addition, the reaction mixture was stirred at room temperature for six hours and poured into water, followed by extraction with ethyl acetate (150 mL). The extract was washed with water, then with a saturated sodium hydrogen carbonate aqueous solution, and with saturated saline, and dried with anhydrous magnesium sulfate. The residue obtained by distilling off the solvent under reduced pressure using a rotary evaporator was purified by silica gel column chromatography (eluent was ethyl acetate), thus obtaining 6-[3',6',9',12',15'-pentaoxa-17'-(4'-*p*-toluenesulfonyloxy)heptadecanyloxy]-2-(4'-dimethylaminostyryl)benzoxazole (780 mg) as an oil.
(2) Under a nitrogen gas stream, 60% sodium hydride (20 mg, 0.5 mmol) was weighed out in a flask, and a solution of 2-(2',2',2'-trifluoroethoxy)ethanol (80 mg, 0.55 mmol) in DMF (0.5 mL) was added dropwise under ice-cooling. After the dropwise addition, the reaction mixture was stirred for 0.5 hours at room temperature. Subsequently, a solution of the 6-[3',6',9',12',15'-pentaoxa-17'-(4'-*p*-toluenesulfonyloxy)heptadecanyloxy]-2-(4'-dimethylaminostyryl)benzoxazole (280 mg, 0.4 mmol) obtained in Step (1) in DMF (0.5 mL) was added dropwise, and the mixture was stirred at room temperature for five hours. The reaction mixture was poured into water, followed by extraction with ethyl acetate (50 mL). The extract was washed with water and then with saturated saline, and dried with anhydrous magnesium sulfate. The residue obtained by distilling off the solvent using a rotary evaporator under reduced pressure was purified by silica gel column chromatography (eluent was ethyl acetate:n-hexane=2:1), thus obtaining 6-(3',6',9',12',15',18',21'-heptaoxa-23',23',23'-trifluorotricosanyloxy)-2-(4'-dimethylaminostyryl)benzoxazole (160 mg) as an oil (mixture of *cis* and *trans* forms).
   ¹HNMR (CDCl₃) : δ3.03 (3H, s), 63.04 (3H, s), 53.6 to 4.0 (26H), δ3.91 (2H, q, J=9Hz), δ4.17 (2H, q, J=5Hz), 6.2 to 7.1 (5H), δ7.4 to 7.9 (4H)
   ¹⁹FNMR (CDCl₃) : δ-75.55 (t, J=9Hz)

### Synthesis Example 2

### Synthesis of 6-(3',6',9',12-,15',18',21',24'-octaoxa-25'-trifluoromethyl-26',26',26'-trifluorohexacosanyloxy)-2-(4'-dimethylaminostyryl)benzoxazole (Compound 2) (not part of the invention)

(1) Under a nitrogen gas stream, 60% sodium hydride (48 mg, 1.2 mmol) was weighed out in a flask, and a solution of 17-(2H-tetrahydropyran-2'-yloxy)-3,6,9,12,15-pentaoxaheptadecan-1-ol (440 mg, 1.2 mmol) in THF (1 mL) was added dropwise under ice-cooling. After the dropwise addition, the reaction mixture was stirred at room temperature for 0.5 hours. Subsequently, a solution of 6-[3'-oxa-5'-(4'-*p*-toluenesulfonyloxy)pentanyloxyl-2-(4'-dimethylaminostyryl)benzoxazole (PTL 3) (522 mg, 1.0 mmol) in THF (2.5 mL) was added dropwise, and the mixture was stirred at room temperature for five hours. The reaction mixture was poured into water, followed by extraction with ethyl acetate (100 mL). The extract was washed with a small amount of water and then with saturated saline, and dried with anhydrous magnesium sulfate. The residue obtained by distilling off the solvent using a rotary evaporator under reduced pressure was purified by silica gel column chromatography (eluent was ethyl acetate), thus obtaining 6-[3',6',9',12',15',18',21'-heptaoxa-23'-(2H-tetrahydropyran-2'-yloxy)tricosanyloxyl-2-(4'-dimethylaminostyryl)benzoxazole (580 mg) as an oil.
   ¹HNMR (CDCl₃): δ1.4 to 1.9 (6H), δ3.03 (6H, s), δ4.63 (1H, t J=3Hz), δ6.6 to 7.1 (5H, arom.), δ7.4 to 7.9 (4H, arom.)
(2) Concentrated hydrochloric acid (0.2 mL) was added to a solution of the 6-[3',6',9',12',15',18',21'-heptaoxa-23'-(2H-tetrahydropyran-2'-yloxy)tricosanyloxy]-2-(4'-dimethylaminostyryl)benzoxazole (560 mg, 0.78 mmol) obtained in Step (1) in ethanol (5 mL) . The reaction mixture was allowed to stand at room temperature for 12 hours, and then neutralized by adding a saturated sodium hydrogen carbonate aqueous solution. After the separation of deposited insoluble matter by filtration, the residue obtained by concentration to dryness under reduced pressure was extracted with dichloromethane. The residue obtained by distilling off dichloromethane using a rotary evaporator was purified by silica gel column chromatography (eluent was methanol:dichloromethane=1:50), thus obtaining 6-(3',6',9',12',15',18',21'-heptaoxa-23'-hydroxytricosanyloxy)-2-(4'-dimethylaminostyryl)benzoxazole (380 mg) as an oil.
   ¹HNMR (CDCl₃) : δ3.03 (6H, s), 63.5 to 3.8 (28H), δ3.90 (2H, m), 54.17 (2H, m), δ6.6 to 7.1 (5H, arom.), δ7.4 to 7.9 (4H, arom.)
(3) Triethylamine (0.14 mL, 1.0 mmol) was added to a solution of the 6-(3',6',9',12',15',18',21'-heptaoxa-23'-hydroxytricosanyloxy)-2-(4'-dimethylaminostyryl)benzoxazole (370 mg, 0.58 mmol) obtained in Step (2) and *p*-toluenesulfonyl chloride (130 mg, 0.68 mmol) in dichloromethane (2 mL), and the mixture was stirred at room temperature for 10 hours. After the completion of the reaction, the reaction mixture was concentrated to dryness under reduced pressure, and the residue was extracted with ethyl acetate. After the ethyl acetate extract was dried with anhydrous magnesium sulfate, the residue obtained by distilling off the solvent using a rotary evaporator under reduced pressure was purified by silica gel column chromatography (eluent was methanol:dichloromethane=1:50), thus obtaining 6-[3',6',9',12',15',18',21'-heptaoxa-23'-(4'-*p-*toluenesulfonyloxy)tricosanyloxy]-2-(4'-dimethylaminostyryl)benzoxazole (420 mg) as an oil.
   ¹HNMR (CDCl₃) : δ2.44 (3H, s), δ3.03 (3H, s), δ3.5 to 3.8 (26H), δ3.89 (2H, m), 54.20 (4H, m), δ6.6 to 7.9 (13H, arom.)
(4) Under a nitrogen gas stream, 60% sodium hydride (30 mg, 0.78 mmol) was weighed out in a flask, and a solution of 1,1,1-3,3,3-hexafluoro-2-propanol (130 mg, 0.78 mmol) in THF (0.5 mL) was added dropwise under ice-cooling. After the dropwise addition, the reaction mixture was stirred at room temperature for 0.5 hours. Subsequently, a solution of the 6-[3',6',9',12',15',18',21'-heptaoxa-23'-(4'-*p-*toluenesulfonyloxy)tricosanyloxy]-2-(4'-dimethylaminostyryl)benzoxazole (125 mg, 0.156 mmol) obtained in Step (3) in THF (0.5 mL) was added dropwise, followed by heating at 55°C for one hour while stirring. The reaction mixture was diluted with ethyl acetate (50 mL), and the ethyl acetate solution was washed with a small amount of water and then with saturated saline, and dried with anhydrous magnesium sulfate. The residue obtained by distilling off the solvent using a rotary evaporator under reduced pressure was purified by silica gel column chromatography (eluent was methanol:dichloromethane=1:100), thus obtaining 6-(3',6',9',12',15',18',21',24'-octaoxa-25'-trifluoromethyl-26',26',26'-trifluorohexacosanyloxy)-2-(4'-dimethylaminostyryl)benzoxazole (mixture of *cis* and trans forms) (100 mg) as an oil.
   ¹HNMR (CDCl₃): δ3.03 (6H, s), δ3.5 to 3.8 (26H), δ3.89 (2H, m), δ3.99 (2H, m), δ4.17 (2H, m), δ4.51 (1H, sep. J=6Hz), δ6.6 to 7.1 (5H, arom.), δ7.4 to 7.9 (4H, arom.)
   ¹⁹FNMR (CDCl₃): δ-75.52 (d, J=6Hz)

### Synthesis Example 3

### Synthesis of 6-(3',6',9',12',15',18',21',24',27'-nonaoxa-29',29',29'-trifluorononacosanyloxy)-2-(4'-dimethylaminostyryl)benzoxazole (Compound 3)

Under a nitrogen gas stream, 60% sodium hydride (30 mg, 0.73 mmol) was weighed out in a flask, and a solution of 2-(2',2',2'-trifluoroethoxy)ethanol (105 mg, 0.73 mmol) in THF (0.5 mL) was added dropwise under ice-cooling. After the dropwise addition, the reaction mixture was stirred for one hour at room temperature. Subsequently, a solution of the 6-[3',6',9',12',15',18',21'-heptaoxa-23'-(4'-*p-*toluenesulfonyloxy)tricosanyloxy]-2-(4'-dimethylaminostyryl)benzoxazole (115 mg, 0.146 mmol) obtained in Step (3) of Synthesis Example 2 in THF (0.5 mL) was added dropwise. After the dropwise addition, the reaction mixture was stirred at room temperature for 16 hours. After the completion of the reaction, the reaction mixture was diluted with ethyl acetate (50 mL), the ethyl acetate solution was washed with a small amount of water and then with saturated saline, and dried with anhydrous magnesium sulfate. The residue obtained by distilling off the solvent using a rotary evaporator under reduced pressure was purified by silica gel column chromatography (eluent was methanol:dichloromethane=1:50), thus obtaining 6-(3',6',9',12',15',18',21',24',27'-nonaoxa-29',29',29'-trifluorononacosanyloxy)-2-(4'-dimethylaminostyryl)benzoxazole (mixture of *cis* and *trans* forms) (80 mg) as an oil.
¹HNMR (CDCl₃) : δ3.03 (6H,s), δ3.5 to 3.8 (32H), δ3.88 (2H, m), δ3.91 (2H, q, J=9Hz), δ4.17 (2H, q, J=5Hz), δ6.6 to 7.1 (5H, arom.), δ7.4 to 7.9 (4H, arom.)
¹⁹FNMR (CDCl₃) : δ-75.55 (t, J=9Hz)

### Synthesis Example 4

### Synthesis of 6-(3',6',9',12',15',18',21',24',27',30',33'-undecaoxa-35',35',35'-trifluoropentatriacontanyloxy)-2-(4'-dimethylaminostyryl)benzoxazole (Compound 4) (not part of the invention)

(1) Under a nitrogen gas stream, 60% sodium hydride (70 mg, 1.75 mmol) was weighed out in a flask, and a solution of 11-(2H-tetrahydropyran-2'-yloxy)-3,6,9-trioxaundecan-1-ol (490 mg, 1.75 mmol) in DMF (2 mL) was added dropwise under ice-cooling. After the dropwise addition, the reaction mixture was stirred for one hour at room temperature. Subsequently, a solution of the 6-(3',6',9',12',15'-pentaoxa-17'-(4'-*p-*toluenesulfonyloxy)heptadecanyloxy]-2-(4'-dimethylaminostyryl)benzoxazole (820 mg, 1.17 mmol) obtained in Step (1) of Synthesis Example 1 in DMF (2 mL) was added dropwise. After the dropwise addition, the reaction mixture was stirred at room temperature for 12 hours. The reaction mixture was poured into water, followed by extraction with ethyl acetate. The extract was washed with a small amount of water and then with saturated saline, and dried with anhydrous magnesium sulfate. The residue obtained by distilling off the solvent using a rotary evaporator under reduced pressure was purified by silica gel column chromatography (eluent was methanol:ethyl acetate=1:50), thus obtaining 6-[3',6',9',12',15',18',21',24',27'-nonaoxa-29'-(2H-tetrahydropyran-2'-yloxy)nonacosanyloxy]-2-(4'-dimethylaminostyryl)benzoxazole (514 mg) as an oil.
   ¹HNMR (CDCl₃): δ1.4 to 1.9 (6H), δ3.03 (6H, s), δ4.63 (1H, m), δ6.6 to 7.1 (5H, arom.), δ7.4 to 7.9 (4H, arom.)
(2) Concentrated hydrochloric acid (0.2 mL) was added to a solution of the 6-[3',6', 9',12',15',18',21',24',27'-nonaoxa-29'-(2H-tetrahydropyran-2'-yloxy)nonacosanyloxy]-2-(4'-dimethylaminostyryl)benzoxazole (500 mg, 0.62 mmol) obtained in Step (1) in ethanol (4 mL), and the mixture was allowed to stand at room temperature for 14 hours. The reaction mixture was then neutralized by adding a saturated bicarbonate aqueous solution. After the separation of deposited insoluble matter by filtration, the residue obtained by concentration to dryness under reduced pressure was extracted with dichloromethane. After the extract was dried with anhydrous magnesium sulfate, the residue obtained by distilling off the solvent using a rotary evaporator was purified by silica gel column chromatography (eluent was methanol:dichloromethane=1:50), thus obtaining 6-(3',6',9',12',15',18',21',24',27'-nonaoxa-29'-hydroxynonacosanyloxy)-2-(4'-dimethylaminostyryl)benzoxazole (390 mg) as an oil.
   ¹HNMR (CDCl₃) : δ3.03 (6H, s), δ3.90 (2H, m), δ4.17 (2H, m), δ6.6 to 7.1 (5H, arom.), δ7.4 to 7.9 (4H, arom.)
(3) Triethylamine (0.14 mL, 1.0 mmol) was added to a solution of the 6- (3',6',9',12',15',18',21',24',27'-nonaoxa-29'-hydroxynonacosanyloxy)-2-(4'-dimethylaminostyryl)bensoxazole (380 mg, 0.53 mmol) obtained in Step (2) and *p*-toluenesulfonyl chloride (130 mg, 0.68 mmol) in dichloromethane (2 mL), and the mixture was stirred at room temperature for 21 hours. The reaction mixture was concentrated to dryness under reduced pressure, and the residue was extracted with ethyl acetate. The ethyl acetate extract was dried with anhydrous magnesium sulfate, and then the solvent was distilled off under reduced pressure using a rotary evaporator. The resulting residue was purified by silica gel column chromatography (eluent was ethyl acetate), thus obtaining 6-[3',6',9',12',15',18',21',24',27'-nonaoxa-29'-(4'-*p-*toluenesulfonyloxy)nonacosanyloxy]-2-(4'-dimethylaminostyryl)benzoxazole (420 mg) as an oil.
   ¹HNMR (CDCl₃): δ2.44 (3H, s), δ3.03 (6H, s), 63.89 (2H, m), δ4.12 (2H, q, J=7Hz), δ6.6 to 7.1 (5H, arom.), δ7.34 (2H, d, J=8Hz), δ7.80 (2H, d, J=8Hz), δ7.4 to 7.9 (4H, arom.)
(4) Under a nitrogen gas stream, 60% sodium hydride (95 mg, 2.37 mmol) was weighed out in a flask, and a solution of 2-(2',2',2'-trifluoroethoxy) ethanol (345 mg, 2.40 mmol) in DMF (1 mL) was added dropwise under ice-cooling. After the dropwise addition, the reaction mixture was stirred at room temperature for 0.5 hours. Subsequently, a solution of the 6-[3',6',9',12',15',18',21',24',27'-nonaoxa-29'-(*p-*toluenesulfonyloxy)nonacosanyloxy]-2-(4'-dimethylaminostyryl)benzoxazole (410 mg, 0.47 mmol) obtained in Step (3) in DMF (1 mL) was added dropwise. After the dropwise addition, the reaction mixture was stirred at room temperature for 12 hours. The residue obtained by concentrating the reaction mixture to dryness under reduced pressure using a rotary evaporator was extracted with ethyl acetate. After the extract was washed with a small amount of saturated saline, it was dried with anhydrous magnesium sulfate. The residue obtained by distilling off the solvent using a rotary evaporator was purified by silica gel column chromatography (eluent was methanol:ethyl acetate=1:50), thus obtaining 6-(3',6',9',12',15',18',21',24',27',30',33'-undecaoxa-35',35',35'-trifluoropentatriacontanyloxy)-2-(4'-dimethylaminostyryl)benzoxazole (mixture of *cis* and *trans* forms) (250 mg) as an oil.
   ¹HNMR (CDCl₃) : δ3.03 (6H, s), δ3.90 (2H, m), δ3.91 (2H, q, J=9Hz), δ4.17 (2H, q, J=5Hz), δ6.6 to 7.1 (5H, arom.), δ7.4 to 7.9 (4H, arom.)
   ¹⁹NMR (CDCl₃) : δ-75.56 (t, J=9Hz)

### Synthesis Example 5

### Synthesis of 6-[3',6',9',12',15',18',21',24',27',30',33'-undecaoxa-35'-(3',5'-bis(trifluoromethyl)benzylamino)pentatriacontanyloxy]-2-(4'-dimethylaminostyryl)benzoxazole (Compound 5) (not part of the invention)

(1) Under a nitrogen gas stream, 60% sodium hydride (45 mg, 1.1 mmol) was weighed out in a flask, and a solution of 3,6,9,12,15-pentaoxa-17-(2H-tetrahydropyran-2'-yloxy)heptadecane-1-ol (410 mg, 1.1 mmol) in DMF (1.5 mL) was added dropwise under ice-cooling. After the dropwise addition, the reaction mixture was stirred at room temperature for one hour. Subsequently, a solution of the 6-[3',6',9',12',15'-pentaoxa-17'-(4'-*p-*toluenesulfonyloxy)heptadecanyloxy]-2-(4'-dimethylaminostyryl)benzoxazole (650 mg, 0.93 mmol) obtained in Step (1) of Synthesis Example 1 in DMF (1.5 mL) was added dropwise. After the dropwise addition, the reaction mixture was stirred at room temperature for 16 hours. The residue obtained by concentrating the reaction mixture to dryness under reduced pressure using a rotary evaporator was extracted with ethyl acetate. Insoluble matter was removed by filtration, and after the filtrate was dried with anhydrous magnesium sulfate, the residue obtained by distilling off the solvent under reduced pressure was purified by silica gel column chromatography (eluent was methanol:dichloromethane=1:40), thus obtaining 6-[3',6',9',12',15',18',21',24',27',30',33'-undecaoxa-35'-(2H-tetrahydropyran-2'-yloxy)pentatriacontanyloxy]-2-(4'-dimethylaminostyryl)benzoxazole (580 mg).
   ¹HNMR (CDCl₃) : δ1.4 to 1.9 (6H), δ3.03 (6H, s), δ4.63 (1H, t, J=3Hz), δ6.6 to 7.1 (5H, arom.), δ7.4 to 7.9 (4H, arom.)
(2) Concentrated hydrochloric acid (0.2 mL) was added to a solution of the 6-[3',6',9',12',15',18',21',24',27',30',33'-undecaoxa-35'-(2H-tetrahydropyran-2'-yloxy)pentatriacontanyloxy]-2-(4'-dimethylaminostyryl)benzoxazole (570 mg, 0.64 mmol) obtained in Step (1) in ethanol (4 mL), and the mixture was allowed to stand at room temperature for three hours. The reaction mixture was neutralized by adding a saturated sodium hydrogen carbonate aqueous solution. Deposited insoluble matter was removed by filtration, and the residue obtained by concentrating the filtrate to dryness under reduced pressure was extracted with dichloromethane. The residue obtained by distilling off the solvent using a rotary evaporator was purified by silica gel column chromatography (eluent was methanol:dichloromethane=1:40), thus obtaining 6-(3',6',9',12',15',18',21',24',27',30',33'-undecaoxa-35'-hydroxypentatriacontanyloxy)-2-(4'-dimethylaminostyryl)benzoxazole (380 mg) as an oil.
   ¹HNMR (CDCl₃) : δ3.03 (6H, s), δ3.90 (2H, m), δ4.18 (2H, m), δ6.6 to 7.1 (5H, arom.), δ7.4 to 7.9 (4H, arom.)
(3) Triethylamine (0.13 mL, 0.93 mmol) was added to a solution of the 6-(3',6',9',12',15',18',21',24',27',30',33'-undecaoxa-35'-hydroxypentatriacontanyloxy)-2-(4'-dimethylaminostyryl)benzoxazole (370 mg, 0.46 mmol) obtained in Step (2) and p-toluenesulfonyl chloride (115 mg, 0.60 mmol) in dichloromethane (2 mL), and the mixture was stirred at room temperature for 18 hours. The reaction mixture was concentrated to dryness under reduced pressure, and the resulting residue was extracted with ethyl acetate. After the separation of insoluble matter by filtration, the residue obtained by concentrating the filtrate to dryness was purified by silica gel column chromatography (eluent was methanol:dichloromethane=1:50), thus obtaining 6-[3',6',9',12',15',18',21',24',27',30',33'-undecaoxa-35'-(4'-*p*-toluenesulfonyloxy)pentatriacontanyloxy]-2-(4'-dimethylaminostyryl)benzoxazole (370 mg) as an oil.
   ¹HNMR (CDCl₃) : δ2.45 (3H, s), δ3.03 (6H, s), δ6.6 to 7.9 (13H, arom.)
(4) Potassium carbonate (310 mg, 2.24 mmol) was added to a solution of the 6-[3',6',9',12',15',18',21',24',27',30',33'-undecaoxa-35'-(4'-p-toluenesulfonyloxy)pentatriacontanyloxyl-2-(4'-dimethylaminostyryl)benzoxazole (360 mg, 0.37 mmol) obtained in Step (3) and 3,5-bis(trifluoromethyl)benzylamine (460 mg, 1.89 mmol) in DMF (3.5 mL), and the mixture was heated at 90°C for nine hours. After the reaction mixture was cooled to room temperature, ethyl acetate (50 mL) was added, and insoluble matter was removed by filtration. The residue obtained by concentrating the filtrate to dryness was purified by silica gel column chromatography (eluent was methanol:dichloromethane=1:30), thus obtaining 6-[3',6',9',12',15',18',21',24',27',30',33'-undecaoxa-35'-(3',5'-bis(trifluoromethyl)benzylamino)pentatriacontanyloxyl-2-(4'-dimethylaminostyryl)benzoxazole (mixture of *cis* and *trans* forms) (250 mg) as an oil.
   ¹HNMR (COCl₃): δ2.81 (2H, t, J=5Hz), δ3.03 (6H, s), δ3.89 (2H, m), δ3.94 (2H, s), δ4.17 (2H, m), δ6.6 to 7.1 (5H, arom.), δ7.76 (1H, s), δ7.83 (2H, s), δ7.4 to 7.9 (4H, arom.)

### Synthesis Example 6

### Synthesis of 6-(3',6',9',12',15',18',21',24',27',30',33',36',39'-tridecaoxa-41',41',41'-trifluorohentetracontanyloxy)-2-(4'-dimethylaminostyryl)benzoxazole (Compound 6) (not part of the invention)

Under a nitrogen gas stream, 60% sodium hydride (15 mg, 0.38 mmol) was weighed out in a flask, and a solution of 2-(2',2',2'-trifluoroethoxy)ethanol (55 mg, 0.38 mmol) in DMF (0.2 mL) was added dropwise under ice-cooling. After the dropwise addition, the reaction mixture was stirred at room temperature for one hour. Subsequently, a solution of the 6-[3',6',9',12',15',18',21',24',27',30',33'-undecaoxa-35'-(4'-*p-*toluenesulfonyloxy)pentatriacontanyloxy]-2-(4'-dimethylaminostyryl)benzoxazole (73 mg, 0.076 mmol) obtained in Step (3) of Synthesis Example 5 in DMF (0.2 mL) was added dropwise, and the reaction mixture was stirred at room temperature for 21 hours. The reaction mixture was concentrated under reduced pressure, and the residue was extracted with ethyl acetate. The ethyl acetate was distilled off using a rotary evaporator under reduced pressure, and then the residue obtained by distilling off the DMF using a vacuum pump was purified by silica gel column chromatography (eluent was methanol:dichloromethane=1:50), thus obtaining 6-(3',6',9',12',15',18',21',24',27',30',33',36',39'-tridecaoxa-41',41',41'-trifluorohentetracontanyloxy)-2-(4'-dimethylaminostyryl) benzoxazole (mixture of *cis* and *trans* forms) (44 mg) as an oil.
¹HNMR (CDCl₃): δ3.03 (6H, s), δ3.73 (2H, q, J=9Hz), δ4.17 (2H, q, J=4.5Hz), δ6.6 to 7.1 (5H, arom.), δ7.4 to 7.9 (4H, arom.)

### Synthesis Example 7

### Synthesis of (2E)-N-[2'-(3',4'-dihydroxyphenyl)ethyl]-3-[4'-hydroxy-3'-(3',6',9',12',15',18',21'-heptaoxa-23',23',23'-trifluorotricosanyloxy)phenyl]-2-propenamide (Compound 7) (not part of the invention

(1) Under a nitrogen gas stream, 60% sodium hydride (1.20 g, 30 mmol) was weighed out in a flask, and a solution of 2-(2',2',2'-trifluoroethoxy) ethanol (3.31 g, 23 mmol) in DMF (5 mL) was added dropwise under ice-cooling. After the dropwise addition, the reaction mixture was stirred at room temperature for one hour. Subsequently, a solution of 1-p-toluenesulfonyloxy-17-(2H-tetrahydropyran-2'-yl)oxy-3,6,9,12,15-pentaoxaheptadecane (5.98 g, 11.5 mmol) in DMF (10 mL) was added dropwise, and the mixture was stirred at room temperature for 18 hours. The residue obtained by concentrating the reaction mixture to dryness was dissolved in ethyl acetate, and the ethyl acetate solution was washed with a small amount of saturated saline, and dried with anhydrous magnesium sulfate. The residue obtained by distilling off the solvent using a rotary evaporator under reduced pressure was purified by silica gel column chromatography (eluent was methanol:dichloromethane=1.50), thus obtaining 1,1,1-trifluoro-23-(2H-tetrahydropyran-2'-yl)oxy-3,6,9,12,15,18,21-heptaoxatricosane (4.76 g) as an oil.
   ¹HNMR (CDCl₃): δ3.91 (2H, q, J=9Hz), δ4.63 (1H, m)
   ¹⁹FNMR (CDCl₃): δ-75.56 (t, J=9Hz)
(2) Concentrated hydrochloric acid (0.1 mL) was added to a solution of the 1,1,1-trifluoro-23-(2H-tetrahydropyran-2'-yl)oxy-3,6,9,12,15,18,21-heptaoxatricosane (4.74 g, 9.6 mmol) obtained in Step (1) in ethanol (60 mL), and the mixture was stirred at room temperature for 16 hours. The mixture was neutralized by adding a saturated sodium hydrogen carbonate solution, and the residue obtained by concentration to dryness was extracted with dichloromethane. The residue (3.87 g, about 9.5 mmol) obtained by distilling off the solvent under reduced pressure was dissolved in dichloromethane (30 mL), and p-toluenesulfonyl chloride (2.35 g, 12.3 mmol) was added thereto. Triethylamine (2.5 mL, 18 mmol) was further added, and the reaction mixture was stirred at room temperature for 15 hours according to Step (3) of Synthesis Example 2. The reaction mixture was washed with a small amount of saturated saline, and then dried with anhydrous magnesium sulfate. The residue obtained by distilling off the solvent under reduced pressure was purified by silica gel column chromatography (eluent was methanol:dichloromethane=1:50), thus obtaining 1,1,1-trifluoro-23-*p*-toluenesulfonylexy-3,6,9,12,15,18,21-heptaoxatricosane (4.87 g) as an oil.
   ¹HNMR (CDCl₃) : δ2.45 (3H, s), δ3.91 (2H, q, J=9Hz), δ7.34 (2H, d, J=8Hz), δ7.80 (2H, d, J=8Hz)
   ¹⁹FNMR (CDCl₃) : δ-75.55 (t, J=9Hz)
(3) The 1,1,1-trifluoro-23-p-toluenesulfonyloxy-3,6,9,12,15,18,21-heptaoxatricosane (2.20 g) obtained in Step (2) and ammonium bromide (0.77 g) were heated while stirring in DMF (9.0 mL) at 80 to 85°C for six hours. The residue obtained by concentrating the reaction mixture under reduced pressure was extracted with ethyl acetate, and the extract was washed with a small amount of water and saturated saline. The extract was then dried with anhydrous magnesium sulfate. The residue obtained by distilling off the solvent under reduced pressure was purified by silica gel column chromatography (eluent was methanol:dichloromethane=1:100), thus obtaining 1,1,1-trifluoro-23-bromo-3,6,9,12,15,18,21-heptaoxatricosane (1.71 g) as an oil.
(4) Potassium carbonate (1.0 g) and sodium iodide (50 mg) were added to a solution in which 4-benzyloxy-3-hydroxybenzaldehyde (0.82 g) and the 1,1,1-trifluoro-23-bromo-3,6,9,12,15,18,21-heptaoxatricosane (1.71 g) obtained in Step (3) were dissolved in acetone (20 mL), and the mixture was stirred at room temperature for 46 hours. After the separation of insoluble matter by filtration, the residue obtained by concentrating the filtrate was extracted with ethyl acetate. The extract was washed with a small amount of water and saturated saline, and then dried with anhydrous magnesium sulfate. Under reduced pressure, the residue obtained by distilling off the solvent was purified by silica gel column chromatography (eluent was methanol:dichloromethane=1:100), thus obtaining 4-benzyloxy-3-(3',6',9',12',15',18',21'-heptaoxa-23',23',23'-trifluoro tricosanyloxy)-benzaldehyde (1.97 g) as an oil.
   ¹HNMR (CDCl₃) : δ3.90 (2H, q, J=9Hz), δ5.22 (2H, s), δ9.83 (1H, s)
   ¹⁹FNMR (CDCl₃) : δ-75.54 (t, J=9Hz)
(5) The 4-benzyloxy-3-(3',6',9',12',15',18',21'-heptaoxa-23',23',23'-trifluorotricosanyloxy)-benzaldehyde (1.97 g) obtained in Step (4) was dissolved in ethanol (16 mL). Ethyl orthoformate (1.60 mL) and DL camphor-10-sulfonic acid (74 mg) were added to the resulting solution, and the mixture was stirred at room temperature for two hours. The reaction mixture was added to a saturated sodium hydrogen carbonate aqueous solution, followed by extraction with ethyl acetate. The extract was washed with saturated saline and dried with anhydrous magnesium sulfate, and then the residue obtained by distilling off the solvent under reduced pressure was purified by silica gel column chromatography (eluent was ethyl acetate:hexane=1:1), thus obtaining 4-benzyloxy-3-(3',6',9',12',15',18',21'-heptaoxa-23',23',23'-trifluorotricosanyloxy)-α,α-diethoxytoluene (2.01 g) as an oil.
   ¹HNMR (CDCl₃) : δ1.23 (6H, t, J=7Hz), δ3.91 (2H, q, J=9Hz), δ5.11 (2H, s), δ5.41 (1H, s), δ6.88 (1H, d, J=8Hz), δ6.97 (1H, dd, J=8Hz, 2Hz), δ7.06 (1H, d, J=2Hz), δ7.3-7.5 (5H)
   ¹⁹FNMR (CDCl₃): δ-75.55 (t, J=9Hz)
(6) 10% Palladium carbon (200 mg) was added to a solution in which the 4-benzyloxy-3-(3',6',9',12',15',18',21'-heptaoxa-23',23',23'-trifluoro tricosanyloxy)-α,α-diethoxytoluene (2.01 g) obtained in Step (5) was dissolved in methanol (40 mL). In hydrogen flow, the mixture was stirred at room temperature for 10 hours. After separation of the catalyst by filtration, 1M-hydrochloric acid (1 mL) was added to a solution in which acetone (6 mL) was dissolved in the residue obtained by concentrating the filtrate to dryness, and the mixture was allowed to stand at room temperature for one hour. The reaction mixture was diluted with ethyl acetate, washed with a saturated sodium hydrogen carbonate aqueous solution, and then with saturated saline, and dried with anhydrous magnesium sulfate. The residue obtained by distilling off the solvent under reduced pressure was purified by silica gel column chromatography (eluent was methanol:dichloromethane=1:100), thus obtaining 4-hydroxy-3-(3',6',9',12',15',18',21'-heptaoxa-23',23',23'-trifluorotricosanyloxy)-benzaldehyde (1.42 g) as an oil.
   ¹HNMR (CDCl₃) : δ3.90 (2H, q, J=9Hz), δ7.00 (1H, d, J=8Hz), δ7.4-7.5 (2H), δ9.79 (1H, s)
   ¹⁹FNMR (CDCl₃) : δ-75.55 (t, J=9Hz)
(7) The 4-hydroxy-3-(3',6',9',12',15',18',21'-heptaoxa-23',23',23'-trifluorotricosanyloxy)-benzaldehyde (710 mg) obtained in Step (6) was dissolved in 7 mL of pyridine. Malonic acid (840 mg) and piperidine (2.3 mL) were added to the solution, and the mixture was heated while stirring to 55 to 60°C. The reaction mixture was ice-cooled and adjusted to pH 2 by adding 2M-hydrochloric acid, followed by extraction with ethyl acetate. A saturated sodium hydrogen carbonate aqueous solution was added to the ethyl acetate solution to extract carbonic acid, and the aqueous solution was washed with diethyl ether. After 2M-hydrochloric acid was added to the aqueous solution to adjust the pH to 2, extraction with ethyl acetate was conducted. The extract was washed with saturated saline, and dried with anhydrous magnesium sulfate. The solvent was then distilled off under reduced pressure, thus obtaining 4-hydroxy-3-(3',6',9',12',15',18',21'-heptaoxa-23',23',23'-trifluorotricosanyloxy)-trans-cinnamic acid (620 mg) as an oil.
   ¹HNMR (CDCl₃) : δ3.90 (2H, q, J=9Hz), δ6.25 (1H, d, J=16Hz), δ6.91 (1H, d, J=8Hz), δ7.12 (1H, dd, J=8Hz, 2Hz), δ7.27 (1H, d, J=2Hz), δ7.68 (1H, d, J=16Hz)
   ¹⁹FNMR (CDCl₃) : δ-75.52 (t, J=9Hz)
(8) A solution of the 4-hydroxy-3-(3',6',9',12',15',18',21'-heptaoxa-23',23',23'-trifluorotricosanyloxy-trans-cinnamic acid (480 mg) obtained in Step (7), 1-hydroxybenzotriazole (230 mg), and 1-ethyl-3-(3'-dimethylaminopropyl) carbodiimide hydrochloride (320 mg) in DMF (2 mL) was ice-cooled, and dopamine hydrochloride (320 mg) was added thereto while stirring. A solution of triethylamine (0.7 mL) in dichloromethane (2 mL) was further added thereto, and the mixture was stirred at room temperature for 24 hours. The reaction mixture was poured into water, followed by extraction with ethyl acetate. The extract was sequentially washed with water, 1M-hydrochloric acid, a saturated sodium hydrogen carbonate aqueous solution, and saturated saline, and dried with anhydrous magnesium sulfate. The residue obtained by distilling off the solvent under reduced pressure was purified by silica gel column chromatography (eluent was methanol: dichloromethane=1:50), thus obtaining (2E)-N-[2'-(3',4'-dihydroxyphenyl)ethyl]-3-[4'-hydroxy-3'-(3',6',9',12',15',18',21'-heptaoxa-23',23',23'-trifluorotricosanyloxy)phenyl]-2-propenamide (290 mg) as an oil.
   ¹HNMR (CDCl₃) : δ3.87 (2H, q, J=9Hz), δ6.19 (1H, d, J=16Hz), δ6.57 (1H, dd, J=8Hz, 2Hz), δ6.75 (1H, d, J=2Hz), δ6.81 (1H, d, J=8Hz), δ6.84 (1H, d, J=8Hz), δ6.9-7.1 (2H), δ7.46 (1H, d, J=16Hz)
   ¹⁹FNMR (CDCl₃) : δ-75.50 (t, J=9Hz)

Compounds 8 and 9 used in the Test Examples below have the structures shown below.

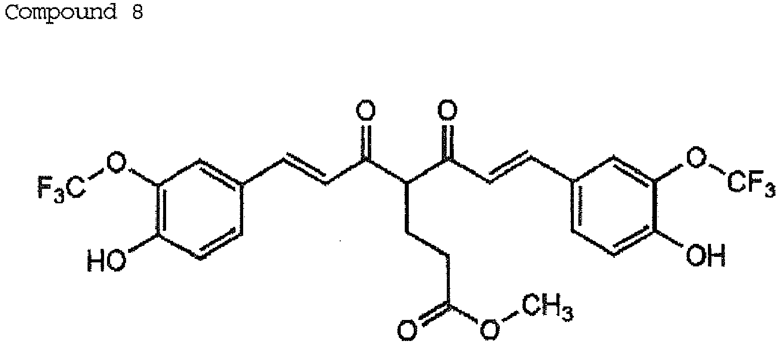

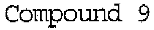

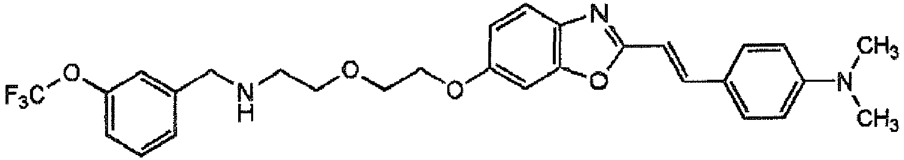

### Test Example 1

### ¹⁹F-MR Image Measurement

Compound 1 was weighed out in an amount of 10 mg, and 0.125 ml of 80% Tween-80 was added thereto, followed by dissolution using a glass rod under heating. Subsequently, 0.875 ml of physiological saline was added thereto to prepare a solution for injection (10 mg/ml). The solution was injected in a total amount of 200 mg/kg at 0.2 kg/ml/min to the tail vein of each of the genetically modified model mice of Alzheimer's disease (APP/PS1) and the normal wild mice (Wild), which had been subjected to pentobarbital sodium anesthesia. As the genetically modified model mice of Alzheimer's disease (APP/PS1), amyloid precursor protein and presenilin 1 double transgenic mice (APP/PS1, purchased from Jackson Laboratory and bred) were used.

After the completion of the injection, under anesthesia, the head of each mouse was continuously evaluated every hour using an MRI apparatus, and all data were added to form images. The brain image of ¹⁹F-MRI was obtained according to the chemical shift imaging method (CSI method). As the MRI apparatus, a 7T Unity Inova MR Scanner (produced by Varian Medical System) was used.

Each of the genetically modified model mice of Alzheimer's disease (APP/PS1) and normal wild mice (Wild) were injected with Compound 1. Fig. 1 shows MRI brain images obtained by performing the measurement in a three-hour period, beginning five hours after injection. Figs. 1A and 1C are ¹⁹F-MRI images, and Figs. 1B and ID are composite photos of ¹⁹F-MRI and ¹H-MRI images. In the ¹⁹F-MR image of the genetically modified model mice of Alzheimer's disease shown in the upper row, signals presumed to be senile plaques were detected inside of the brain; however, such signals were not detected in the wild mouse shown in the lower row.

### Test Example 2

### Brain Tissue Staining

The mouse brain subjected to the ¹⁹F-MRI scan performed in Test Example 1 was collected and fixed for two days in a 4% formalin solution. The brain was then transferred to a 15% sucrose solution for cryoprotection, and a slice with a thickness of 20 µm was produced using a cryostat. Subsequently, the slice was reacted overnight at 4°C with a rabbit anti-human amyloid β polyclonal antibody (produced by Immuno-Biological Laboratory Co., Ltd., 0.2 µg/ml). Further, the specimen was washed with PBS-T for 10 minutes three times, and then reacted at room temperature for four hours with an Alexa 647 anti-rabbit IgG antibody (Molecular Probes, 500-fold dilution). After the specimen was washed with PBS-T for 10 minutes three times, counterstaining was performed for one minute using cresyl violet. The specimen was further washed with distilled water, and was mounted with glycerol. The binding property of the compound to senile plaques was observed using an inverted fluorescence microscope. The compound image was measured with a DAPI filter set, and the amyloid β protein image was measured with a Cy5 filter set. The same procedure was performed for the normal mice.

Fig. 2 shows the brain stain images of the resulting genetically modified model mice of Alzheimer's disease. Fig. 2A shows a fluorescence image of the compound observed with a DAPI filter set, and Fig. 2B shows a β amyloid antibody stain image. Spots of amyloid β, which forms senile plaques, which were observed in Fig. 2B, correspond to those of the fluorescence stain image of the compound observed in Fig. 2A. This indicates that the compound bound to the senile plaques.

### Test Example 3

### Analysis of Binding Property with Aβ Oligomers or Aβ Fibrils Using QCM Apparatus

Aβ oligomers or Aβ fibrils were fixed to the sensor portion of the QCM apparatus, and immersed in PBS in a glass container. Subsequently, Compound 8 or Compound 1 was added thereto to a final concentration of 10 µM, and a frequency change was recorded from immediately after the addition.

Fig. 3 shows the obtained results. Fig. 3A shows the frequency change obtained when Compound 8 was added, whereas Fig. 3B shows the frequency change obtained when Compound 1 was added. When Compound 8 was added, significant frequency reduction was observed from the sensor to which Aβ oligomers were fixed (Oligomer) and the sensor to which Aβ fibrils were fixed (Fibrillar) compared to the sensor to which nothing was fixed (Control). In contrast, when Compound 8 was added, although frequency reduction was observed from the sensor to which Aβ fibrils were fixed (Fibrillar), nearly the same frequency change was observed from the sensor to which Aβ oligomers were fixed (Oligomer) and the sensor to which nothing was fixed (Control). The above results indicate that although Compound 8 has a property of binding to both Aβ oligomers and Aβ fibrils, Compound 1 binds to Aβ fibrils, but does not bind to Aβ oligomers.

### Test Example 4

### ¹⁹F-MR Imaging Test of Compound 1 and Compound 8

Compound 1 and Compound 8 were each weighed out in an amount of 10 mg, and 80% Cremophor-EL was added to each compound in an amount of 0.25 ml, followed by dissolution under heating. Subsequently, 0.75 ml of physiological saline was added to each resultant to prepare solutions for injection (10 mg/ml). Each solution was individually injected in a total amount of 200 mg/kg at 0.2 kg/ml/min into the vein of an APP/PS1 mouse subjected to pentobarbital sodium anesthesia.

Three hours after the completion of the injection, the mice were euthanized with an overdose of pentobarbital sodium, and ¹⁹F-MR signals from the mouse heads were measured using an MR apparatus. In the measurement, single-pulse measurement for obtaining an NMR spectrum was carried out for 10 minutes. Then, 50-minute measurement for imaging according to the chemical shift imaging method (CSI method) was repeated, and data were added together after the completion of the measurement to form images.

Fig. 4 shows the obtained results. Fig. 4A shows the NMR spectrum of a 16-month-old APP/PS1 mouse. Fig. 4B shows the ¹H-MR images of 16-month-old APP/PS1 mouse, ¹⁹F-MR images of Compound 8, ¹⁹F-MR images of Compound 1, and images showing ¹⁹F-MR signal difference between Compound 8 and Compound 1. The upper row shows horizontal images, and the lower row shows sagittal images. In the NMR spectrum, Compound 8 and Compound 1 showed completely different peaks. By using the chemical shift of each peak, separate imaging of Compound 8 and Compound 1, which were injected at the same time, was successful. Further, by calculating image differences between Compound 8 and Compound 1, differences in signal distribution or strength of these two compounds were successfully depicted. Fig. 4C shows the images obtained from an 11-month-old APP/PS1 mouse. Even in a younger mouse, separate imaging of Compound 8 and Compound 1, which were injected at the same time, was also successful.

### Test Example 5

### Comparison of MR images between Compound 1, Compound 7, and Compound 9

Compound 1, Compound 7, and Compound 9 were separately dissolved in DMSO to have a concentration of 10 mM. Each mouse subjected to pentobarbital sodium anesthesia was fixed to a brain stereotaxic apparatus, and the scalp was cut to expose the skull. Subsequently, using a drill, holes were made 0.1 mm anterior to, and 2.3 mm to the right and to the left of the bregma. A Hamilton syringe needle filled with a pharmaceutical agent was then inserted into each hole to a depth of 2.5 mm from the arachnoid membrane to inject 2 µL of the pharmaceutical agent. After being allowed to stand for 10 minutes, the needle was removed. Compound 9 was injected into the left hole, and Compound 1 or Compound 7 was injected into the right hole. Immediately thereafter, the mice were euthanized with an overdose of pentobarbital sodium. The imaging test of the compounds was performed using the MR apparatus. For imaging, data obtained by the 20-minute measurement according to the CSI method were used.

Fig. 5 shows the obtained results. Fig. 5A shows the MR images of a mouse head in which Compound 9 was injected into the left brain and Compound 1 was injected into the right brain, and Fig. 5B shows the MR images of a mouse head in which Compound 9 was injected into the left brain and Compound 7 was injected into the right brain. The upper row of each figure shows horizontal or coronal ¹H MR and ¹⁹F MR images, and the lower row shows combined ¹H MR and ¹⁹F MR images. Although ¹⁹F MR signals of Compound 9 were not detected from the left brain into which Compound 9 was injected, ¹⁹F MR signals of Compound 1 or Compound 7 were detected from the right brain into which Compound 1 or Compound 7 was injected (see the arrow); thus, imaging was successful.

### Industrial Applicability

Since the compound of the present invention has a high binding specificity to and a high detectivity for amyloid β proteins that have B-sheet structures, it functions as an MRI contrast medium. Thus, the compound of the present invention can be used for antemortem noninvasive diagnosis of amyloid accumulation diseases, such as Alzheimer's disease, using an MRI apparatus that is commonly used in medical institutions.

Since the compound of the present invention has high detectivity, it can also be used for antemortem noninvasive diagnosis of diseases other than Alzheimer's disease, i.e., diseases wherein tau protein, α-synuclein, or TDP-43 accumulates.

## Claims

1. A compound represented by formula (I), formula (XI), or a salt thereof, 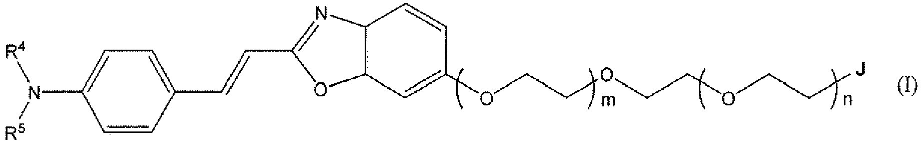 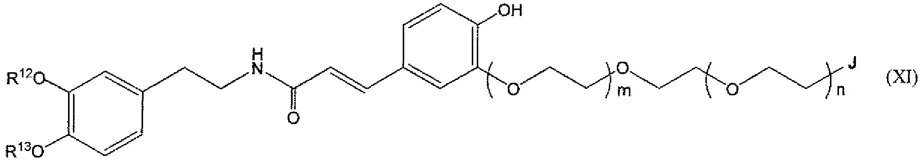 wherein R⁴ and R⁵ represent C₁₋₆ alkyl; R¹² and R¹³ are each independently a hydrogen atom or C₁₋₆ alkyl; m is an integer of 0 to 6; n is an integer of 1 to 5; and J is the following group 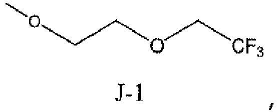 provided that 5 ≤ m + n ≤ 7.

2. A diagnostic imaging agent for a disease in which an amyloid β protein accumulates, the diagnostic imaging agent comprising the compound or a salt thereof according to claim 1 as an active ingredient.

3. The diagnostic imaging agent according to claim 2, wherein the disease in which an amyloid β protein accumulates is Alzheimer's disease.

4. The diagnostic imaging agent according to claim 2 or 3, wherein diagnostic imaging is MRI.

5. A staining agent for an amyloid β protein in tissue, including the brain, or a senile plaque in tissue, including the brain, the staining agent comprising the compound or a salt thereof according to claim 1 as an active ingredient.

6. A method for staining an amyloid β protein in tissue or a senile plaque in tissue, the method comprising using the staining agent according to claim 5.

7. A diagnostic imaging agent for use in detection of an amyloid β oligomer, the diagnostic imaging agent comprising in combination
(A) the compound or a salt thereof according to claim 1, and
(B) a curcumin derivative represented by formula (2) or a salt thereof, 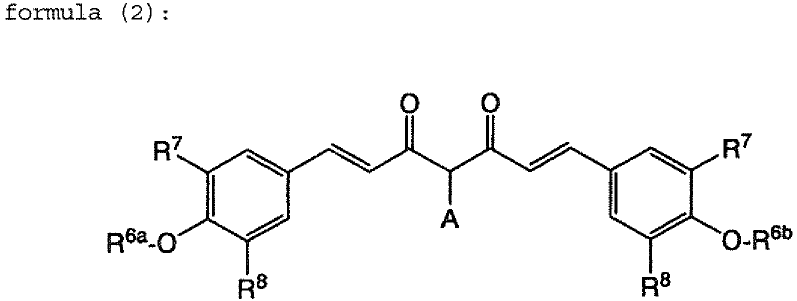
wherein R^{6a} and R^{6b} are each independently a hydrogen atom, alkyl, acetyl, or methoxycarbonyl; R⁷s are each independently a fluorine atom, CHF₂-, CF₃-, CHF₂O-, or CF₃O-; R⁸s are each independently a hydrogen atom or a fluorine atom; A is alkyl, cyano, carboxyl, alkoxycarbonyl, or R⁹-(CH₂)_{q}-, R⁹ is hydroxy, carboxy, cyano, acetyloxy, alkoxycarbonyl, alkoxyalkoxy, hydroxyalkoxy, or CONR¹⁰R¹¹; R¹⁰ and R¹¹ are each independently a hydrogen atom or alkyl; and q is an integer of 1 to 5.

8. A method for detecting an amyloid β oligomer, the method comprising using the diagnostic imaging agent according to claim 7.

## Patentansprüche

1. Verbindung, dargestellt durch die Formel (I), Formel (XI) oder ein Salz davon, 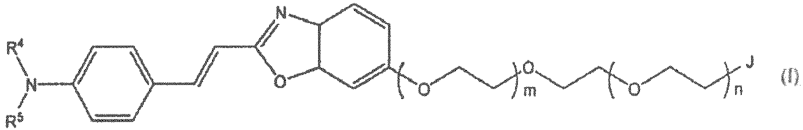 wobei R⁴ und R⁵ C₁₋₆-Alkyl darstellen; R¹² und R¹³ jeweils unabhängig ein Wasserstoffatom oder C₁₋₆-Alkyl sind; m eine ganze Zahl von 0 bis 6 ist; n eine ganze Zahl von 1 bis 5 ist; und J die folgende Gruppe ist 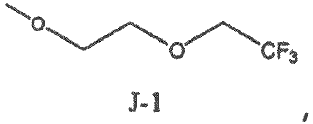 vorausgesetzt, dass 5 ≤ m + n ≤ 7.

2. Bildgebungs-Diagnostikum für eine Erkrankung, bei der sich ein Amyloid-β-Protein ansammelt, wobei das Bildgebungs-Diagnostikum die Verbindung oder ein Salz davon nach Anspruch 1 als Wirkbestandteil umfasst.

3. Bildgebungs-Diagnostikum nach Anspruch 2, wobei die Erkrankung, bei der sich ein Amyloid-β-Protein ansammelt, die Alzheimer-Erkrankung ist.

4. Bildgebungs-Diagnostikum nach Anspruch 2 oder 3, wobei das Bildgebungs-Diagnostikum eine MRT ist.

5. Färbemittel für ein Amyloid-β-Protein in einem Gewebe, einschließlich des Gehirns, oder einer Senil-Plaque in einem Gewebe, einschließlich des Gehirns, wobei das Färbemittel die Verbindung oder ein Salz davon nach Anspruch 1 als Wirkbestandteil umfasst.

6. Verfahren zum Färben eines Amyloid-β-Proteins in einem Gewebe oder einer Senil-Plaque in einem Gewebe, wobei das Verfahren die Verwendung des Färbemittels nach Anspruch 5 umfasst.

7. Bildgebungs-Diagnostikum zur Verwendung beim Nachweis eines Amyloid-β-Oligomers, wobei das Bildgebungs-Diagnostikum in Kombination umfasst
(A) die Verbindung oder ein Salz davon nach Anspruch 1, und
(B) ein Curcuminderivat, dargestellt durch Formel (2) oder ein Salz davon, 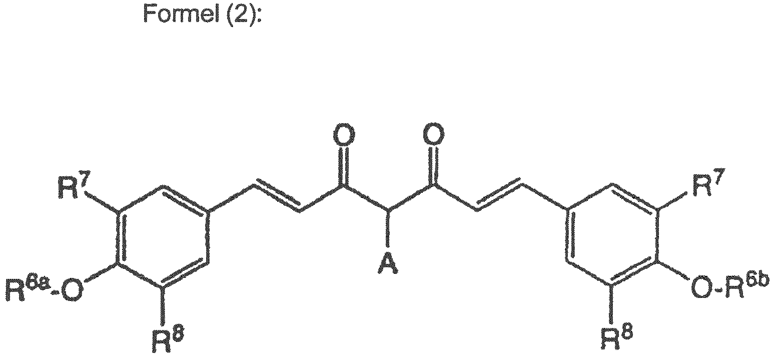
wobei R^{6a} und R^{6b} jeweils unabhängig ein Wasserstoffatom, Alkyl, Acetyl, oder Methoxycarbonyl sind; R⁷ jeweils unabhängig voneinander ein Fluoratom, CHF₂-, CF₃-, CHF₂O-, oder CF₃O- sind; R⁸ jeweils unabhängig ein Wasserstoffatom oder ein Fluoratom sind; A Alkyl, Cyano, Carboxyl, Alkoxycarbonyl, oder R⁹-(CH₂)_{q}- ist; R⁹ Hydroxy, Carboxy, Cyano, Acetyloxy, Alkoxycarbonyl, Alkoxyalkoxy, Hydroxyalkoxy oder CONR¹⁰R¹¹ ist; R¹⁰ und R¹¹ jeweils unabhängig ein Wasserstoffatom oder Alkyl sind; und q eine ganze Zahl von 1 bis 5 ist.

8. Verfahren zum Nachweisen eines Amyloid-β-Oligomers, wobei das Verfahren die Verwendung des Bildgebungs-Diagnostikums nach Anspruch 7 umfasst.

## Revendications

1. Composé représenté par la formule (I), la formule (XI) ou un sel de celui-ci, 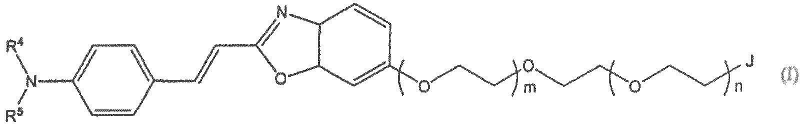 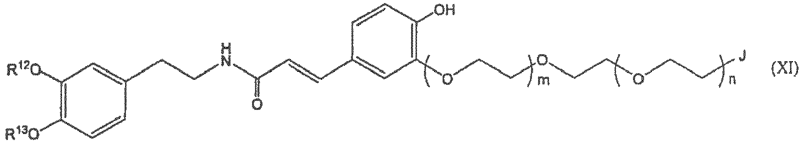 dans lesquelles R⁴ et R⁵ représentent un alkyle en C₁₋₆ ; R¹² et R¹³ sont chacun indépendamment un atome d'hydrogène ou un alkyle en C₁₋₆ ; m est un entier de 0 à 6 ; n est un entier de 1 à 5 ; et J est le groupe suivant 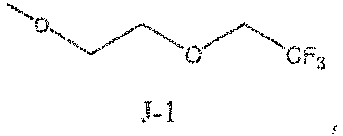 à condition que 5 ≤ m + n ≤ 7.

2. Agent d'imagerie de diagnostic pour une maladie dans laquelle une protéine β-amyloïde s'accumule, l'agent d'imagerie de diagnostic comprenant le composé selon la revendication 1 ou un sel de celui-ci en tant qu'ingrédient actif.

3. Agent d'imagerie de diagnostic selon la revendication 2, dans lequel la maladie dans laquelle une protéine β-amyloïde s'accumule est la maladie d'Alzheimer.

4. Agent d'imagerie de diagnostic selon la revendication 2 ou 3, dans lequel l'imagerie de diagnostic est une IRM.

5. Agent de coloration pour une protéine β-amyloïde dans un tissu, incluant le cerveau, ou une plaque sénile dans un tissu, incluant le cerveau, l'agent de coloration comprenant le composé selon la revendication 1 ou un sel de celui-ci en tant qu'ingrédient actif.

6. Procédé de coloration d'une protéine β-amyloïde dans un tissu ou d'une plaque sénile dans un tissu, le procédé comprenant l'utilisation de l'agent de coloration selon la revendication 5.

7. Agent d'imagerie de diagnostic pour utilisation dans la détection d'un oligomère β-amyloïde, l'agent d'imagerie de diagnostic comprenant en combinaison
(A) le composé selon la revendication 1 ou un sel de celui-ci, et
(B) un dérivé de curcumine représenté par la formule (2) ou un sel de celui-ci, 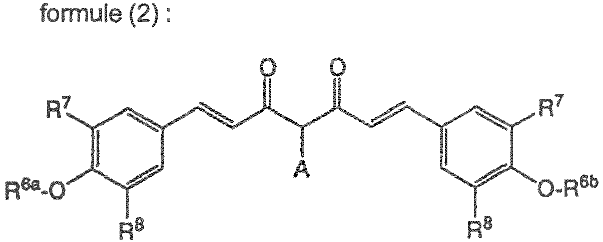
dans laquelle R^{6a} et R^{6b} sont chacun indépendamment un atome d'hydrogène, alkyle, acétyle ou méthoxycarbonyle ; les R⁷ sont chacun indépendamment un atome de fluor, CHF₂-, CF₃-, CHF₂O-, ou CF₃O- ; les R⁸ sont chacun indépendamment un atome d'hydrogène ou un atome de fluor ; A est alkyle, cyano, carboxyle, alcoxycarbonyle, ou R⁹-(CH₂)_{q}- ; R⁹ est hydroxy, carboxy, cyano, acétyloxy, alcoxycarbonyle, alcoxyalcoxy, hydroxyalcoxy, ou CONR¹⁰R¹¹ ; R¹⁰ et R¹¹ sont chacun indépendamment un atome d'hydrogène ou alkyle ; et q est un entier de 1 à 5.

8. Procédé de détection d'un oligomère β-amyloïde, le procédé comprenant l'utilisation de l'agent d'imagerie de diagnostic selon la revendication 7.
